# EUROPÄISCHE PATENTSCHRIFT

(11) **EP 1 833 321 B1**
(45) Veröffentlichungstag und Bekanntmachung des Hinweises auf die Patenterteilung: **28.01.2015**
(21) Anmeldenummer: 05812324.1
(22) Anmeldetag: 02.12.2005
(51) Int. Cl.: A45D 19/02, A61K 8/00, A61Q 5/08, A61Q 5/10

(54) **ABGABESYSTEM FÜR FARBMODIFIZIERENDE MITTEL**
DISPENSING SYSTEM FOR COLOUR-MODIFYING AGENTS
SYSTEME DISTRIBUTEUR POUR DES TEINTURES

(30) Priorität: 17.12.2004 DE 102004061468; 08.06.2005 DE 102005026533
(43) Veröffentlichungstag der Anmeldung: 19.09.2007
(73) Patentinhaber: Henkel AG & Co. KGaA, 40589 Düsseldorf (DE)
(72) Erfinder: KLAMP, Susanne, 41539 Dormagen (DE); FIGLEWICZ, Monika, 41470 Neuss (DE); ELBERG, Emilie, 40479 Düsseldorf (DE); PAULI, Kristin, 42119 Wuppertal (DE); SEILER, Martina, 47228 Duisburg (DE); SCHAAL, Dorothee, 08015 Barcelona (ES)
(86) Internationale Anmeldenummer: PCT/EP2005/012939
(87) Internationale Veröffentlichungsnummer: WO 2006/027279

(56) Entgegenhaltungen:
- EP-A- 1 557 110
- DE-U1- 20 218 244
- FR-A- 2 647 093
- US-A- 3 194 426
- US-A- 4 209 027
- US-A- 5 954 213
- US-A- 6 012 462
- US-B1- 6 688 314

## Beschreibung

Die vorliegende Erfindung betrifft ein Abgabesystem für haarfarbverändernde Mittel, enthaltend mindestens zwei Behälter und mindestens zwei Austrittsöffnungen, sowie ein Verfahren zur Strähnchenfärbung unter zur Hilfenahme dieses Abgabesystems.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Der US 6012462 A ist beispielsweise eine Abgabevorrichtung zur gleichzeitigen Abgabe von mehreren unterschiedlichen Haarfärbezubereitungen auf das Haar zu entnehmen. Dazu besitzt die dort beschriebene Abgabevorrichtung mehrere separate Vorratsbehälter für jeweils unterschiedliche Haarfärbezubereitungen, wobei jeder Behälter mit eigenen Abgabeöffnungen versehen ist. Diese Abgabevorrichtung ist jedoch aufgrund ihres komplexen Gesamtaufbaus mit einer Drucklufteinrichtung zum Ausbringen der Haarfärbezubereitungen aus den einzelnen Behältern nicht für einen Gebrauch im privaten Umfeld des Nutzers geeignet.

Aus der US 4209027 A ist darüber hinaus eine Abgabevorrichtung für ein Haarbehandlungsmittel bekannt, die als Quetschspender ausgebildet ist. Die dortige Abgabevorrichtung besitzt zwei unterschiedliche Ausgabeöffnungen, die je nach Bedarf für die jeweilige Anwendung ausgewählt werden können. Im Einzelnen ist eine erste Ausgabeöffnung in eine Applikationsspitze integriert, während die zweite Ausgabeöffnung innerhalb einer Applikationsbürste angeordnet ist. Dadurch lässt sich das Haarbehandlungsmittel zwar Anwendungsgerecht aufbringen jedoch ist stets nur die Anwendung eines einzelnen Haarbehandlungsmittels möglich.

Aus der FR 2 647 093 A ist ein Abgabesystem für haarfarbverändernde Mittel gemäss der Präambel des Anspruchs 1 bekannnt.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvoprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muss üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

In jüngster Zeit ist der Wunsch der Verbraucher nach sogenannten Multi-Ton-Haarfarben gewachsen. Insbesondere die Applikation von Strähnchen in mehreren verschiedenen Farben nebeneinander erfreut sich großer Beliebtheit. Ein derartiger Vorgang ist derzeit mit einem erheblichen Aufwand verbunden und wird üblicherweise nur in Friseursalons angeboten.

Überraschenderweise wurde nunmehr gefunden, dass mehrfarbige Strähnchen mit einem speziellen Abgabesystem in einem Anwendungsschritt auf einfache Weise erhalten werden können. Somit kann die mehrfarbige Strähnchenapplikation in einem Schritt unter Anwendung der vorliegenden Erfindung auch für die Heimanwendung zur Verfügung gestellt werden.

Gegenstand der vorliegenden Erfindung ist daher ein Abgabesystem für haarfarbverändernde Mittel, welches mindestens zwei lösbar koppelbare Behälter aufweist, wobei jeder Behälter mindestens eine Austrittsöffnung aufweist, wobei die Behälter unterschiedliche haarfarbverändernde Mittel enthalten und die Austrittsöffnungen voneinander räumlich so isoliert angeordnet sind , dass es möglich ist, die unterschiedlichen haarfarbverändernden Mittel gleichzeitig an voneinander beabstandeten Orten aufzutragen. Dazu weisen die Behälter jeweils abgeflachte längere Außenseiten auf und sind mit diesen abgeflachten längeren Außenseiten zueinander angeordnet. Dabei weist der Rand einer Austrittsöffnung jeweils zwei, auf gegenüberliegenden Seiten der

Austrittsöffnung angeordnete und in Fließrichtung abstehende Vorsprünge auf, welche im Wesentlichen in einer Ebene parallel zu den kürzeren Außenseiten der Behälter angeordnet sind, wobei zwischen den Vorsprüngen Borsten und/oder ein Auftragsschwämmchen zum gleichmäßigen Auftragen der Mittel angeordnet sind.

Es spielt dabei erfindungsgemäß keine wesentliche Rolle, welcher Art die mindestens zwei verschiedenen haarfarbverändernden Mittel sind. Erfindungsgemäß bevorzugt sind Blondiermittel gegebenenfalls mit Boosterzusatz, oxidative Färbemittel sowie Tönungsmittel. Erfindungsgemäß sind alle Kombinationen dieser haarfarbverändernden Mittel denkbar. So können beispielsweise
- zwei verschiedene oxidative Färbemittel,
- zwei verschiedene Tönungsmittel,
- zwei verschieden starke Blondiermittel,
- ein Blondiermittel und ein oxidatives Färbemittel,
- ein Blondiermittel und ein Tönungsmittel oder
- ein oxidatives Färbemittel und ein Tönungsmittel
gemeinsam in dem erfindungsgemäßen Abgabesystem für haarfarbverändernde Mittel konfektioniert sein.

Im Rahmen einer ersten bevorzugten Ausführungsform enthält mindestens ein Behälter ein Blondiermittel.

Blondiermittel im Sinne der vorliegenden Erfindung sind haarfarbverändernde Mittel, die die Haare in einen Farbton überführen, der heller ist als das Ausgangshaar.

Die erfindungsgemäßen Blondiermittel enthalten bevorzugt als Oxidationsmittel Wasserstoffperoxid oder eine Anlagerungsverbindung von Wasserstoffperoxid an anorganische oder organische Verbindungen, wie beispielsweise Natriumperborat, Natriumpercarbonat, Natriumpercarbamid, Polyvinylpyrrolidon, Harnstoffperoxid und Melaminperoxid.

Die Blondierwirkung kann bevorzugt durch sogenannte "Booster" gesteigert werden. Dies sind in der Regel feste Peroxoverbindungen, die keine Anlagerungsprodukte von Wasserstoffperoxid an andere Komponenten darstellen. Die Auswahl dieser Peroxoverbindungen unterliegt prinzipiell keinen Beschränkungen; übliche, dem Fachmann bekannte Peroxoverbindungen sind beispielsweise Ammoniumperoxodisulfat, Kaliumperoxodisulfat, Natriumperoxodisulfat, Ammoniumpersulfat, Kaliumpersulfat, Natriumpersulfat, Kaliumperoxodiphosphat, Percarbonate wie Magnesiumpercarbonat und Peroxide wie Bariumperoxid. Unter diesen Peroxoverbindungen, die auch in Kombination eingesetzt werden können, sind erfindungsgemäß die anorganischen Verbindungen bevorzugt. Besonders bevorzugt sind die Peroxodisulfate, insbesondere Ammoniumperoxodisulfat.

Die Peroxoverbindungen sind in den erfindungsgemäßen Blondiermitteln bevorzugt in Mengen von 1-30 Gew.-%, insbesondere in Mengen von 5-20 Gew.-%, enthalten.

Als weitere wichtige Komponente enthalten die erfindungsgemäßen Blondiermittel ein Alkalisierungsmittel, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall- und Erdalkalimetallhydroxide, -carbonate, -hydrogencarbonate, -hydroxycarbonate, - silikate, insbesondere -metasilikate, sowie Alkaliphosphate verwendet werden. In einer bevorzugten Ausführungsform enthalten die erfindungsgemäßen Blondiermittel mindestens zwei unterschiedliche Alkalisierungsmittel. Dabei können Mischungen beispielsweise aus einem Hydroxycarbonat und einem Metasilikat bevorzugt sein.

Die Zubereitungen enthalten Alkalisierungsmittel bevorzugt in Mengen von 1-25 Gew.-%, insbesondere 1,5-20 Gew.-%.

Die erfindungsgemäßen Zubereitungen, enthaltend wesentliche Komponenten der Blondiermittel, können als Feststoff vorliegen oder können in einen geeigneten wässrigen, alkoholischen oder wässrig-alkoholischen Träger eingearbeitet sein. Zum Zwecke der Blondierung sind solche Träger beispielsweise Cremes, Emulsionen, Gele, Pasten, oder andere Zubereitungen, die für die Anwendung auf dem Haar geeignet sind. Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Zubereitungen können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Wird die Zubereitung als Pulver konfektioniert, enthalten sie in der Regel zusätzlich eine Komponente zur Entstaubung der feinpulverisierten Formulierung. Solche Entstaubungsmittel sind üblicherweise Öle, flüssige Wachse, Etherderivate. In der Druckschrift WO 00/30596 werden bei 25°C flüssige Lösemittel, ausgewählt aus der Gruppe der Kohlenwasserstoffe, der Alkohole, der Ester sowie der Ketone, wie z.B. 3-Methoxybutanol, Benzylalkohol, 1,2-Propandiol, Hexanol, Cyclohexanon, Propylencarbonat und Ethyldiglykol offenbart, die sich zur Entstaubung eignen und in den erfindungsgemäßen Zubereitungen enthalten sein können.

Die folgende Rahmenrezeptur steht exemplarisch für ein erfindungsgemäßes Blondiermittel ohne dieses darauf zu beschränken:

| | |
|---|---|
| Fettalkohol / -säure | 13 - 17% |
| Tensid (z.B. Fettalkoholethersulfat, APG) | 10 - 15% |
| Emulgator (z.B. Fettalkoholethoxylate) | 0,8 - 1,2% |
| Reduktionsmittel (z.B. Natriumsulfit; Ascorbinsäure) | 1 - 2% |
| Alkalisierungsmittel (z.B. Ammoniak / MEA) | 6 - 10% |
| Komplexbildner und Wasserglas | ca. 1 % |
| Parfüm | 0,2 - 0,5% |
| Wasser | ad 100 |

Es hat sich als erfindungsgemäß besonders bevorzugt erwiesen, wenn die Blondiermittel, die in dem erfindungsgemäßen Abgabesystem zum Einsatz kommen, frei von Verdickungsmitteln, insbesondere frei von polymeren Verdickungsmitteln, formuliert werden. Darüber hinaus hat sich der Einsatz von Fettalkoholalkoxylaten in Mengen von 0,8 bis 1,2 Gew.% als besonders bevorzugt erwiesen.

Ferner zeichnen sich die erfindungsgemäßen Blondiermittel der o. g. Rahmenrezeptur dadurch aus, dass sie
- auch bei hohen Konzentrationen von Salzen stabil sind,
- eine optimale Viskosität für das Ausbringverhalten aufweisen sowie
- sich auf dem Haar durch eine gute Benetzung und ein geringes Tropfen auszeichnen.

In dem Fall, dass in einem der Behälter des erfindungsgemäßen Abgabesystem ein Blondiermittel zum Einsatz kommt, befindet sich bei der Verkaufsware im Rahmen einer bevorzugten Ausführungsform die verdünnte Wasserstoffperoxidlösung bereits in dem Behälter des Abgabesystem. Wohingegen die zweite - üblicherweise feste oder pastenförmige - Zubereitung mit festen Oxidationsmitteln unmittelbar vor der Anwendung hinzugegeben wird. Diese Mischung wird dann mittels des erfindungsgemäßen Abgabesystem auf das Haar aufgebracht. Die Konzentration dieser Wasserstoffperoxid-Lösung wird einerseits von den gesetzlichen Vorgaben und andererseits von dem gewünschten Effekt bestimmt; in der Regel werden 6- bis 12-prozentige Lösungen in Wasser verwendet. Die Mengenverhältnisse von Blondiermittel und Wasserstoffperoxid-Lösung liegen dabei üblicherweise im Bereich von 1:1 bis 1:2, wobei ein Überschuss an Wasserstoffperoxid-Lösung insbesondere dann gewählt wird, wenn eine ausgeprägte Blondierwirkung erwünscht ist.

Im Rahmen einer zweiten bevorzugten Ausführungsform enthält mindestens ein Behälter ein oxidatives Färbemittel.

Unter oxidativen Färbemittel sind erfindungsgemäß haarfarbverändernde Mittel zu verstehen, die durch Oxidation von Oxidationsfarbstoffvorprodukten eine dauerhafte Färbung der Fasern bewirken.

Hinsichtlich der in den erfindungsgemäßen Färbemitteln einsetzbaren Farbstoffvorprodukte unterliegt die vorliegende Erfindung keinerlei Einschränkungen. Die erfindungsgemäßen Färbemittel können als Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.

Im Rahmen einer ersten bevorzugten Ausführungsform der vorliegenden Erfindung enthalten die erfindungsgemäßen Mittel mindestens ein Oxidationsfarbstoffvorprodukt vom Entwickler- und/oder Kupplertyp.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 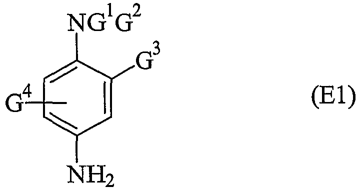 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 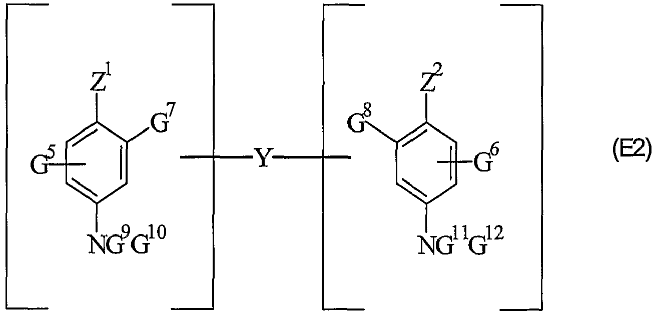 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 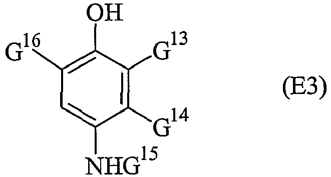 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 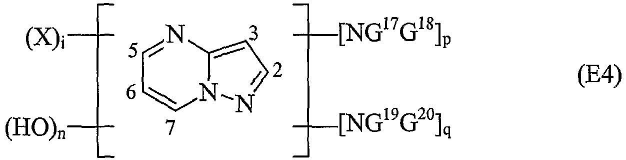 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

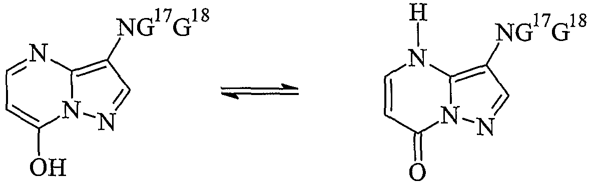

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform enthalten die erfindungsgemäßen oxidativen Färbemittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resor-cinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Die erfindungsgemäßen Färbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

In einer weiteren Ausführungsform der vorliegenden Erfindung enthalten die Färbemittel als Oxidationsfarbstoffvorprodukt mindestens eine Vorstufe eines naturanalogen Farbstoffs. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (IIa), 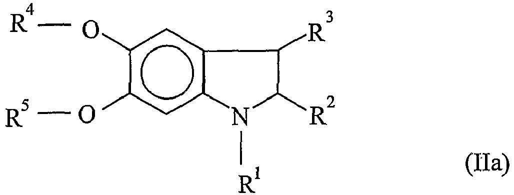 in der unabhängig voneinander
- R¹ steht für Wasserstoff, eine C₁-C₄-Alkylgruppe oder eine C₁-C₄-Hydroxy-alkyl-gruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbon-säure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIb), 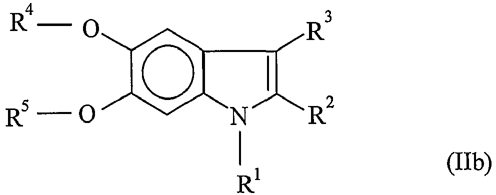 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Färbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Neben den Oxidationsfarbstoffvorprodukten können die oxidativen Färbemittel zur weiteren Nuancierung auch direktziehende Farbstoffe enthalten. Hinsichtlich der erfindungsgemäß bevorzugten direktziehenden Farbstoffe sei an dieser Stelle auf die später im Text folgenden Ausführungen explizit Bezug genommen.
Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbemitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die folgende Rahmenrezeptur steht exemplarisch für ein erfindungsgemäßes oxidatives Färbemittel ohne dieses darauf zu beschränken:

| | |
|---|---|
| Fettalkohol / -säure | 13 - 17% |
| Tensid (z.B. Fettalkoholethersulfat, APG) | 10 - 15% |
| Emulgator (z.B. Fettalkoholethoxylate) | 0,8 - 1,2 % |
| Reduktionsmittel (z.B. Natriumsulfit; Ascorbinsäure) | 1 - 2% |
| Alkalisierungsmittel (z.B. Ammoniak / MEA) | 6 - 10% |
| Komplexbildner und Wasserglas | ca. 1 % |
| Parfüm | 0,2 - 0,5 % |
| Farbstoffvorproduktkombination | bis 5% |
| Wasser | ad 100 |

Es hat sich als erfindungsgemäß besonders bevorzugt erwiesen, wenn die oxidativen Färbemittel, die in dem erfindungsgemäßen Abgabesystem zum Einsatz kommen, frei von Verdickungsmitteln, insbesondere frei von polymeren Verdickungsmitteln, formuliert werden. Darüber hinaus hat sich der Einsatz von Fettalkoholalkoxylaten in Mengen von 0,8 bis 1,2 Gew.-% als besonders bevorzugt erwiesen. Ferner zeichnen sich die erfindungsgemäßen Färbemittel der o.g. Rahmenrezeptur dadurch aus, dass sie
- auch bei hohen Konzentrationen von Salzen (dabei bis zu 5 Gew.-% an Farbstoff(vorprodukt)en) stabil sind,
- eine optimale Viskosität für das Ausbringverhalten aufweisen sowie
- sich auf dem Haar durch eine gute Benetzung und ein geringes Tropfen auszeichnen.

In dem Fall, dass in einem der Behälter des erfindungsgemäßen Abgabesystems ein oxidatives Färbemittel zum Einsatz kommt, befindet sich bei der Verkaufsware im Rahmen einer bevorzugten Ausführungsform die verdünnte Wasserstoffperoxidlösung bereits in dem Behälter des Abgabesystem für haarfarbverändernde Mittel. Wohingegen die zweite Zubereitung, enthaltend die Oxidationsfarbstoffvorprodukte unmittelbar vor der Anwendung hinzugegeben wird. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem alkalischen Milieu.

In einer dritten bevorzugten Ausführungsform enthält mindestens ein Behälter ein Tönungsmittel.

Unter Tönungsmittel sind erfindungsgemäß haarfarbverändernde Mittel zu verstehen, die einen oder mehrere direktziehende Farbstoffe enthalten. Die direktziehenden Farbstoffe sind vorzugsweise ausgewählt aus den Nitrophenylendiaminen, den Nitroaminophenolen, den Azofarbstoffen, den Anthrachinonen oder den Indophenolen.

Besonders bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner kann es erfindungsgemäß bevorzugt sein, dass die Tönungsmittel mindestens einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die mindestens einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

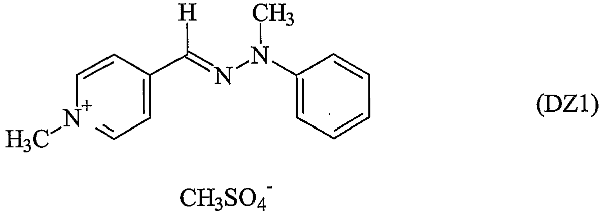

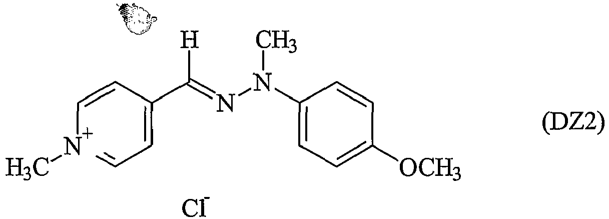

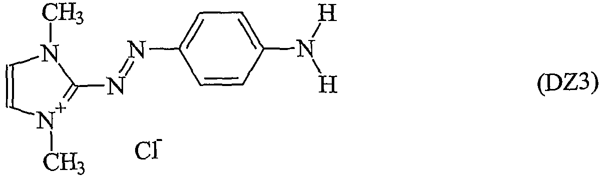

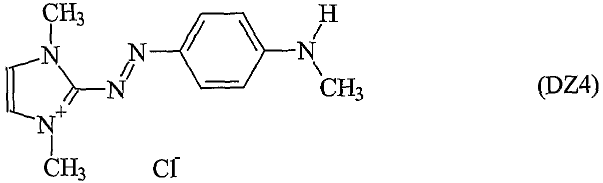

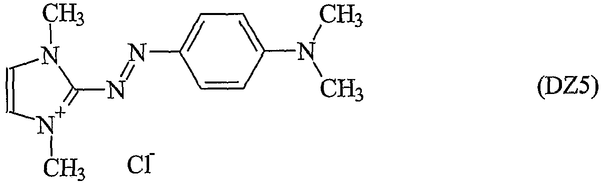

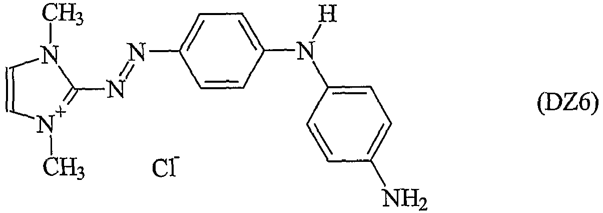

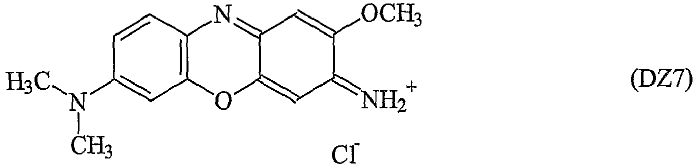

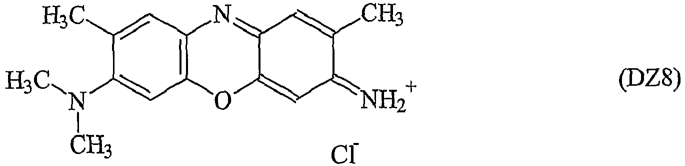

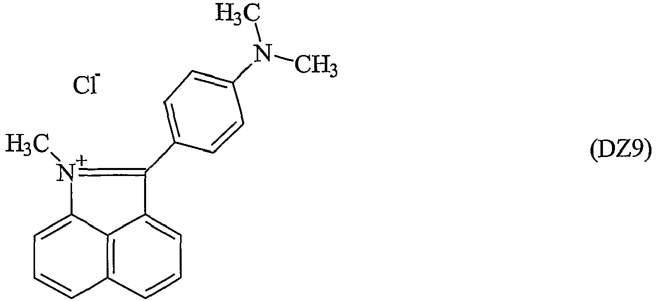

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Weiterhin können die erfindungsgemäßen Zubereitungen auch in der Natur vorkommende direktziehende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Die erfindungsgemäßen Mittel enthalten die direktziehenden Farbstoffe bevorzugt in einer Menge von 0,01 bis 20 Gew.-%, bezogen auf die gesamten Anwendungszubereitung in dem jeweiligen Behälter.

Es ist nicht erforderlich, dass die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haartönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

In dem Fall, dass in einem der Behälter des erfindungsgemäßen Abgabesystems ein Tönungsmittel zum Einsatz kommt, so befindet sich gemäß der vorliegenden Erfindung das gesamte Tönungsmittel bereits in der Verkaufsware. Eine Zugabe von einer oder mehreren weiteren Komponenten ist aber prinzipiell nicht ausgeschlossen. So kann beispielsweise unmittelbar vor der Anwendung eine haarkonditionierende und/oder eine farbintesivierende Komponente zu dem Tönungsmittel hinzugegeben werden.

Das erfindungsgemäße Abgabesystem für haarfarbverändernde Mittel umfasst mindestens zwei Behälter, wobei jeder dieser Behälter mindestens eine Austrittsöffnung aufweist. Die Austrittsöffnungen der Behälter bzw. die Behälter selbst sind so angeordnet, dass die Austrittsöffnungen voneinander isoliert sind.

Im Rahmen einer ganz besonders bevorzugten Ausführungsform umfasst das erfindungsgemäße Abgabesystem für haarfarbverändernde Mittel genau zwei Behälter, wobei jeder dieser Behälter mindestens eine Austrittsöffnung aufweist.

Die mindestens zwei Behälter können alle für diesen Zweck denkbaren Geometrien aufweisen. Behälterformen die zylinderförmig gestaltet sind, sind bevorzugt. Es ist aber auch denkbar, dass Behälter in Form von Gestalten, wie beispielsweise Figuren und/oder Tieren, zum Einsatz kommen. Weiterhin kann sich selbstverständlich aus der Verknüpfung der Behälter miteinander sowie mit den Austrittsöffnungen eine abweichende Geometrie ergeben.

Die Behälter, insbesondere wenn sie zylinderförmig sind, können beispielsweise eine ovale, eine dreieckige, eine rechteckige oder eine trapezförmige Grundfläche aufweisen. Es sollen auch Behälter mit solchen Grundflächen umfasst sein, die sich von den oben genannten Grundflächen durch geringe Abwandlung, wie beispielsweise eine Abrundung der Ecken, ergeben.

Erfindungsgemäß sollen prinzipiell alle räumlichen Anordnungen der Behälter umfasst sein.

In einer nicht erfindungsgemässen Ausführungsform kann das Abgabesystem einstückig gestaltet sein.

In einer weiteren, nicht erfindugsgemäßen Ausführungsform umfasst das Abgabesystem für haarfarbverändernde Mittel zwei Behälter, die eine runde beziehungsweise quadratische Grundfläche mit abgerundeten Ecken aufweisen. Die Behälter sind im Rahmen dieser Ausführungsform bevorzugt nebeneinander angeordnet.

Im Rahmen einer weiteren Ausführungsform der vorliegenden Erfindung weisen die Behälter eine ovale beziehungsweise eine rechteckige Grundfläche mit abgerundeten Ecken auf. Im Rahmen der Erfindung sind die Behälter derart angeordnet, dass die jeweils längeren Außenseiten zueinander hinzeigen oder aneinander anliegen.

Die vorzugsweise zwei Behälter können weiterhin geometrisch so geformt sein, dass sie aneinander oder ineinander gesteckt und damit mechanisch miteinander fest oder fest lösbar verbunden werden können.

Dies kann beispielsweise dadurch erreicht werden, dass ein Behälter den anderen zumindest teilweise in seinem Umfang umgreift und diesen in definierter Position festklemmt. Eine exakte Positionierung der Behälter zueinander kann dabei beispielsweise dadurch herbeigeführt werden, dass einer der Behälter an seiner Oberfläche Rastnoppen oder Rastnasen aufweist, die in entsprechende Vertiefungen oder Aussparungen des zweiten Behälters eingreifen und ein Verrutschen der Behälter zueinander verhindern.

In einer weiteren Ausführungsform können die Behälter auch so ausgebildet sein, dass einer der Behälter an seiner Oberfläche mindestens eine Nut und der andere Behälter mindestens eine darin eingreifende Feder aufweist, so dass die Behälter auf diese Weise zusammengesteckt werden können.

Diese Ausführungsform mit Nut und Feder kann in vorteilhafter Weise dadurch weitergebildet sein, dass die Nut oder die Feder Rastnoppen und dementsprechend die Feder oder die Nut dazu korrespondierende Rastvertiefungen aufweist. Diese Einrastmittel, also die Rastnoppen und -Vertiefungen, verriegeln die Steckverbindung der beiden miteinander verbundenen Behälter, so dass diese nur mit erhöhter Krafteinwirkung voneinander getrennt werden können.

In einer besonders bevorzugten Ausführungsform können die Behälter als zwei gleich geformte Hälften in einer im Wesentlichen geometrisch als Kegelstumpf ausgebildeten Ausgabesystems ausgebildet sein, wobei der Kegelstumpf an seiner Schmalseite zwei jeweils seitlich der Mittelachse angeordnete Auslassöffnungen aufweist. Jede der Hälften, also der Behälter, weist damit eine konvex geformte Außenseite auf, die eine Hälfte der Kegelstumpfoberfläche bildet, und eine im Wesentlichen plane Außenseite auf, an der die Hälften aneinander anliegen. Jede der Hälften ist dabei so ausgeformt, dass der untere Teil fließend über die gesamte Höhe des Kegelstumpfes in den jeweils aus der Mittelachse zur Seite versetzten Auslass übergeht. Die plane Außenseite einer jeden Hälfte weist weiterhin mindestens ein mechanisches Verbindungselement wie zum Beispiel Nut bzw. Feder auf, so dass die Hälften zusammengesteckt und fest miteinander verbunden werden können.

Weiterhin kann es bevorzugt sein, wenn die mindestens zwei Behälter aus Kunststoff, kunststoffhaltigen Mehrschichtfolien, metallisierten Folien oder Metall hergestellt sind.

Die räumlich isolierten Austrittsöffnungen sind erfindungsgemäß derart angeordnet, dass eine Vermischung der unterschiedlichen haarfarbverändernden Mittel sowohl bei der Lagerung als auch bei der Anwendung auf dem Haar ausgeschlossen ist. Erfindungsgemäß sind die Austrittsöffnungen derart zu gestalten, dass auch eine Rückvermischung im Anschluss an die Anwendung vermieden wird.

Im Rahmen einer Ausführungsform können auf den mindestens zwei isolierten Austrittsöffnungen zwei getrennte Applikatoraufsätze installiert sein. Im Rahmen dieser Ausgestaltungsform ist es bevorzugt, wenn die mindestens zwei Behälter fest-lösbar miteinander verbunden sind und jeder von ihnen einen Applikatoraufsatz trägt. Dabei ist es erfindungsgemäß denkbar, dass zwei gleichartige aber auch zwei unterschiedliche Applikatoraufsätze verwendet werden.

Im Rahmen einer weiteren Ausgestaltungsform können die mindestens zwei räumlich isolierten Austrittsöffnungen auch einen gemeinsamen Applikatoraufsatz mit zwei getrennten Öffnungen tragen. Im Rahmen dieser Ausgestaltungsform ist es bevorzugt, wenn die mindestens zwei Behälter unabhängig voneinander sind und durch den gemeinsamen Applikatoraufsatz zusammengehalten werden. Es kann aber erfindungsgemäß auch denkbar sein, dass die mindestens zwei Behälter bereits miteinander verknüpft sind und der gemeinsame Applikatoraufsatz eine zusätzliche Verbindung herstellt.

Die Austrittsöffnungen und ihr räumliches Umfeld sind erfindungsgemäß derart gestaltet, dass sie zum Auftragen der haarfarbverändernden Mittel auf die Haare geeignet sind. Dazu hat es sich als vorteilhaft erwiesen, wenn der Applikatoraufsatz derart gestaltet ist, dass Borsten, Kämme oder mit Watte umgebene Stäbe rund um die Austrittsöffnung angeordnet sind. Weiterhin kann es erfindungsgemäß bevorzugt sein, wenn nach mindestens einer Austrittsöffnung ein Schwamm angeordnet ist. Ebenso kann es erfindungsgemäß bevorzugt sein, wenn in mindestens eine Austrittsöffnung ein Faserverbund reinreicht. Ferner kann es erfindungsgemäß bevorzugt sein, wenn nach mindestens einer Austrittsöffnung ein Kugelventil angeordnet ist. Weiterhin sind die mindestens zwei Austrittsöffnungen durch Trennwände voneinander getrennt.

Erfindungsgemäß ganz besonders bevorzugt ist eine kreisförmige Anordnung von Borsten rund um die Austrittsöffnung.
Weiterhin kann es bevorzugt sein, wenn jeder der Behälter mehrere nebeneinander angeordnete Austrittsöffnungen aufweist, wobei die Austrittsöffnungen durch nachfolgende Trennwände voneinander getrennt sind. Im Rahmen dieser Ausführungsform sind bevorzugt sein, wenn im Bereich der Austrittsöffnung weitere Applikationshilfen, wie beispielsweise Borsten, ein Schwamm, ein Kamm, ein von Watte umhüllter Stab, ein Kugelventil oder ein Faserverbund angeordnet.

Erfindungsgemäß ist der Applikatoraufsatz dadurch gekennzeichnet,

dass der Rand der Austrittsöffnung an zwei gegenüberliegenden Seiten zwei Vorsprünge aufweist, so dass die Austrittsöffnung zwischen den Vorsprüngen angeordnet ist. Damit kann eine solche Applikatoröffnung auf dem Haar so aufgesetzt werden, dass durch die beiden Vorsprünge eine Haarsträhne von den übrigen Haaren abgeteilt wird und das Mittel genau und ausschließlich auf die erfasste Strähne appliziert wird. Die Vorsprünge können beim Applizieren der Mittel aus dem zugehörigen Behälter auf das Haar auf die Kopfhaut aufgesetzt werden, so dass damit ein minimaler Abstand zwischen der Austrittsöffnung und der Kopfhaut sichergestellt ist. Schließlich ist eine eine solche Applikatorspitze noch dadurch gekennzeichnet, dass der Applikator zwischen den Vorsprüngen Borsten oder ein Auftragsschwämmchen aufweist, um einen gleichmäßigen Auftrag des aufzutragenden Mittels sicherzustellen.

Auch hinsichtlich des Ausbringungsmechanismus soll die vorliegend Erfindung in keiner Weise eingeschränkt sein. So kann die Ausbringung beispielsweise durch Schwerkraft, durch Kapillarkräfte oder durch Druckausbringung erfolgen. Als Beispiele für die Druckausbringung seien an dieser Stelle die Ausübung von Druck auf flexible Wände, der Innendruck (Aerosolausbringung) sowie Pumpmechanismen erwähnt.

Im Rahmen einer nicht erfindungsgemässen Ausführungsform sind in den mindestens zwei räumlich voneinander getrennten Austrittsöffnungen mindestens zwei miteinander verbundene Mascarabürsten als Applikatoraufsatz enthalten, die jeweils in einen Behälter mit einem haarfarbverändernden Mittel tauchen, gemeinsam aus den Austrittsöffnungen genommen werden können, und mittels derer die mindestens zwei unterschiedlichen haarfarbverändernden Mittel nebeneinander auf die Haarsträhnen aufgebracht werden können.

Die in den Behältern des erfindungsgemäßen Abgabesystem für haarfarbverändernde Mittel enthaltenden haarfarbverändernden Mittel können neben den bereits beschriebenen wesentlichen Komponenten natürlich eine Reihe weiterer Komponenten enthalten.

Im Rahmen einer ersten bevorzugten Ausführungsform enthalten die haarfarbverändernden Mittel mindestens einen Pflegestoff.

Ein erfindungsgemäß bevorzugter Pflegestoff ist beispielsweise ein kationisches Tensid. Bevorzugt sind kationische Tenside vom Typ der quarternären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammo-niumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxy-ethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Die kationischen Tenside sind in den erfindungsgemäßen Mitteln bevorzugt in Mengen von 0,05 bis 10 Gew.-%, jeweils bezogen auf die gesamte Anwendungszubereitung in dem jeweiligen Behälter, enthalten. Mengen von 0,1 bis 5 Gew.-% sind besonders bevorzugt.

Ein erfindungsgemäß bevorzugter Pflegestoff ist weiterhin ein pflegendes Polymer.

Eine erste Gruppe der pflegenden Polymere sind die kationischen Polymere. Unter kationischen Polymeren sind erfindungsgemäß Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 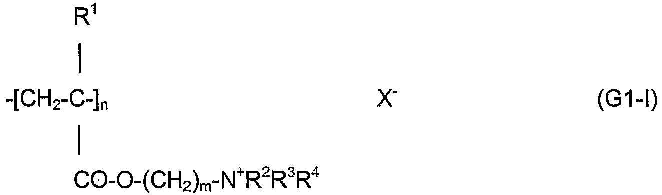 in der R¹= -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylenether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosme-dia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Weiterhin sind kationiserte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Weitere erfindungsgemäß einsetzbare pflegende Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten amphoteren Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (II),

   R¹-CH=CR²-CO-Z-(CₙH₂ₙ)-N⁽⁺⁾R³R⁴R⁵ A⁽⁻⁾ (II)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (III),

   R⁶-CH=CR⁷-COOH (III)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet. Die erfindungsgemäßen Zweikomponentenmittel enthalten die kationischen Polymere bevorzugter Weise in einer Menge von 0,01 bis 5 Gew.-%, insbesondere in einer Menge von 0,1 bis 2 Gew.-%, jeweils bezogen auf die gesamte Anwendungszubereitung in dem jeweiligen Behälter.

Ein erfindungsgemäß bevorzugter Pflegestoff ist ferner ein UV-Filter.

Die erfindungsgemäß geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexyl-salicylat (Homosalate), 2-Hydroxy-4-methoxy-benzophenon (Benzophenone-3; Uvinul^{®}M 40, Uvasorb^{®}MET, Neo Heliopan^{®}BB, Eusolex^{®}4360), 2-Phenylbenzimidazol-5-sulfonsäure und deren Kalium-, Natrium- und Triethanolaminsalze (Phenylbenzimidazole sulfonic acid; Parsol^{®}HS; Neo Hellopan^{®}Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol^{®}1789, Eusolex^{®}9020), α-(2-Oxoborn-3-yliden)-toluol-4-sulfonsäure und deren Salze, ethoxylierte 4-Aminobenzoesäure-ethylester (PEG-25 PABA; Uvinul^{®}P 25), 4-Dimethylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}O18), 4-Methoxyzimtsäure-isopentylester (Isoamyl p-Methoxycinnamate; Neo Heliopan^{®}E 1000), 4-Methoxyzimtsäure-2-ethylhexyl-ester (Octyl Methoxycinnamate; Parsol^{®}MCX, Escalol^{®}557, Neo Heliopan^{®}AV), 2-Hydroxy-4-methoxybenzophenon-5-sulfonsäure und deren Natriumsalz (Benzophenone-4; Uvinul^{®}MS 40; Uvasorb^{®}S 5), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahy-droxybenzophenon (Benzophenone-2; Uvinul^{®}D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethyl-hexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methyl-benzyliden)-D,L-Campher, 3-Benzyliden-campher, 4-Isopropylbenzylsalicylat, 2,4,6-Tri-anilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, dass bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Diese UV-Filter weisen die allgemeine Struktur U - Q auf.

Der Strukturteil U steht dabei für eine UV-Strahlen absorbierende Gruppe. Diese Gruppe kann sich im Prinzip von den bekannten, im Kosmetikbereich einsetzbaren, oben genannten UV-Filtern ableiten, in dem eine Gruppe, in der Regel ein Wasserstoffatom, des UV-Filters durch eine kationische Gruppe Q, insbesondere mit einer quartären Aminofunktion, ersetzt wird.

Verbindungen, von denen sich der Strukturteil U ableiten kann, sind beispielsweise
- substituierte Benzophenone,
- p-Aminobenzoesäureester,
- Diphenylacrylsäureester,
- Zimtsäureester,
- Salicylsäureester,
- Benzimidazole und
- o-Aminobenzoesäureester.

Strukturteile U, die sich vom Zimtsäureamid oder vom N,N-Dimethylamino-benzoesäureamid ableiten, sind erfindungsgemäß bevorzugt.

Die Strukturteile U können prinzipiell so gewählt werden, daß das Absorptionsmaximum der UV-Filter sowohl im UVA(315-400 nm)-, als auch im UVB(280-315nm)- oder im UVC(<280 nm)-Bereich liegen kann. UV-Filter mit einem Absorptionsmaximum im UVB-Bereich, insbesondere im Bereich von etwa 280 bis etwa 300 nm, sind besonders bevorzugt.

Weiterhin wird der Strukturteil U, auch in Abhängigkeit von Strukturteil Q, bevorzugt so gewählt, daß der molare Extinktionskoeffizient des UV-Filters am Absorptionsmaximum oberhalb von 15 000, insbesondere oberhalb von 20000, liegt.

Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so daß der Strukturteil U einen der vier Substituenten des positiv geladenen Stickstoffatomes darstellt. Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.

Vorteilhafterweise hat die Gruppe Q die allgemeine Struktur -(CH₂)_{X}-N⁺R¹R²R³ X⁻, in der x steht für eine ganze Zahl von 1 bis 4, R¹ und R² unabhängig voneinander stehen für C₁₋₄-Alkylgruppen, R³ steht für eine C₁₋₂₂-Alkylgruppe oder eine Benzylgruppe und X⁻ für ein physiologisch verträgliches Anion. Im Rahmen dieser allgemeinen Struktur steht x bevorzugt für die die Zahl 3, R¹ und R² jeweils für eine Methylgruppe und R³ entweder für eine Methylgruppe oder eine gesättigte oder ungesättigte, lineare oder verzweigte Kohlenwasserstoffkette mit 8 bis 22, insbesondere 10 bis 18, Kohlenstoffatomen.

Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyl-dimethylaminobenzamidopropyl-dimethylammoniumtosylat (Escalol^{®} HP 610).

Selbstverständlich umfasst die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter sind in den erfindungsgemäßen Mitteln üblicherweise in Mengen 0,01-5 Gew.-%, bezogen auf die gesamte Anwendungszubereitung in dem jeweiligen Behälter, enthalten. Mengen von 0,1-2,5 Gew.-% sind bevorzugt.

Ein erfindungsgemäß bevorzugte Pflegestoffe sind ebenso Vitamin, Provitamine, Vitaminvorstufen sowie eines derer Derivate.

Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%, bezogen auf die gesamte Anwendungszubereitung in dem jeweiligen Behälter.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß Mitteln bevorzugt in Mengen von 0,05 bis 1 Gew.-%, bezogen auf die gesamte Anwendungszubereitung in dem jeweiligen Behälter, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf die gesamte Anwendungszubereitung in dem jeweiligen Behälter, enthalten. Mengen von 0,1 - 5 Gew.-% sind besonders bevorzugt.
- Vitamin B₆ (Pyridoxin sowie Pyridoxamin und Pyridoxal). Die genannten Verbindungen des Vitamin B₆-Typs sind in den erfindungsgemäßen Mitteln bevorzugt in Mengen von 0,01 - 5 Gew.-%, bezogen auf die gesamte Anwendungszubereitung in dem jeweiligen Behälter, enthalten. Mengen von 0,05 - 1 Gew.-% sind besonders bevorzugt.

Vitamin C (Ascorbinsäure). Vitamin C wird in den erfindungsgemäß verwendeten Mitteln bevorzugt in Mengen von 0,1 bis 3 Gew.-%, bezogen auf die gesamte Anwendungszubereitung in dem jeweiligen Behälter eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf die gesamte Anwendungszubereitung in dem jeweiligen Behälter, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-%, jeweils bezogen auf die gesamte Anwendungszubereitung in dem jeweiligen Behälter enthalten.

Bevorzugt enthalten die erfindungsgemäßen Zweikomponentenmittel Vitamine, Provitamine und Vitaminvorstufen aus den Gruppen A, B, C, E und H.

Panthenol, Pantolacton, Pyridoxin und seine Derivate sowie Nicotinsäureamid und Biotin sind besonders bevorzugt.

Ein erfindungsgemäß bevorzugter Pflegestoff ist weiterhin ein Pflanzenextrakt.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders geeignet sind die Extrakte aus Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Ein erfindungsgemäß bevorzugter Pflegestoff ist ferner eine Carbonsäure.

Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäßen Carbonsäuren können selbstverständlich entlang der Kohlenstoffkette oder des Ringgerüstes substituiert sein. Zu den Substituenten der erfindungsgemäßen Carbonsäuren sind beispielsweise zu zählen C₁-C₈-Alkyl-, C₂-C₈-Alkenyl-, Aryl-, Aralkyl- und Aralkenyl-, Hydroxymethyl-, C₂-C₈-Hydroxyalkyl-,C₂-C₈-Hydroxyalkenyl-, Aminomethyl-, C₂-C₈-Aminoalkyl-, Cyano-, Formyl-, Oxo-, Thioxo-, Hydroxy-, Mercapto-, Amino-, Carboxy- oder Iminogruppen. Bevorzugte Substituenten sind C₁-C₈-Alkyl-, Hydroxymethyl-, Hydroxy-, Amino- und Carboxygruppen. Besonders bevorzugt sind Substituenten in α-Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Als Beispiele für erfindungsgemäße Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I), 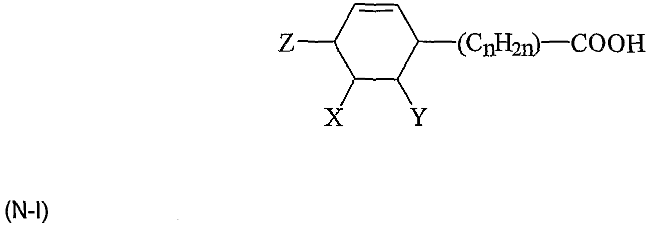 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Dicarbonsäuren der Formel (N-I) sind in der Literatur bekannt. So ist beispielweise der US-Patentschrift 3,753,968 ein Herstellungsverfahren zu entnehmen.

Die Dicarbonsäuren der Formel (N-I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuß vorliegt. Diese Isomerengemische können erfindungsgemäß ebenso wie die reinen Verbindungen eingesetzt werden.

Erfindungsgemäß einsetzbar neben den bevorzugten Dicarbonsäuren gemäß Formel (N-I) sind auch solche Dicarbonsäuren, die sich von den Verbindungen gemäß Formel (N-I) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid^{®} 1550 und Westvaco Diacid^{®} 1595 (Hersteller: Westvaco) erhältlich.

Neben den zuvor beispielhaft aufgeführten erfindungsgemäßen kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt 2-Pyrrolidinon-5-carbonsäure und deren Derivate als Carbonsäure einzusetzen. Besonders bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf die gesamte Anwendungszubereitung in dem jeweiligen Behälter, besonders bevorzugt 0,1 bis 5, und insbesondere 0,1 bis 3 Gew.%.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren einzusetzen. Hierbei hat sich gezeigt, dass neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der ß-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C₁₂-C₁₅-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

Ein erfindungsgemäß bevorzugter Pflegestoff ist ebenso ein Proteinhydrolysat und eines seiner Derivate.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Die Proteinhydrolysate sind in den erfindungsgemäßen Zweikomponentenmitteln in Konzentrationen von 0,01 Gew.% bis zu 20 Gew.%, vorzugsweise von 0,05 Gew.% bis zu 15 Gew.% und ganz besonders bevorzugt in Mengen von 0,05 Gew.% bis zu 5 Gew.%, jeweils bezogen auf die gesamte Anwendungszubereitung in dem jeweiligen Behälter enthalten.

Weiterer erfindungsgemäß bevorzugte Pflegestoffe sind beispielsweise Ectoin oder Ectoinderivate, Allantoin, Taurin und Bisabolol.

Erfindungsgemäß werden unter dem Begriff "Ectoin und Ectoinderivate" Verbindungen der Formel (IV) 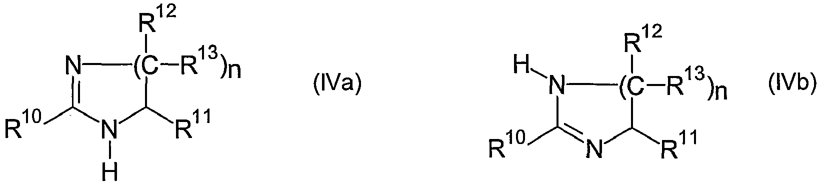 und/oder deren physiologisch verträglichen Salzes und/oder einer isomeren oder stereoisomeren Form verstanden, wobei
R¹⁰ steht für ein Wasserstoffatom, einen verzweigten oder unverzweigten C₁ - C₄-Alkylrest oder einen C₂ - C₄-Hydroxyalkylrest,
R¹¹ steht für ein Wasserstoffatom, eine Gruppierung -COOR¹⁴ oder eine Gruppierung-CO(NH)R¹⁴, wobei R¹⁴ für ein Wasserstoffatom, einen C₁ - C₄-Alkylrest, einen Aminosäurerest, einen Dipeptid- oder einen Tripeptidrest stehen kann,
R¹² und R¹³ stehen unabhängig voneinander für ein Wasserstoffatom, einen C₁ - C₄-Alkylrest oder einer der beiden Reste steht für eine Hydroxygruppe und
n steht für eine ganze Zahl von 1 bis 3.

Geeignete physiologisch verträgliche Salze der allgemeinen Verbindungen gemäß der Formel (IVa) oder (IVb) sind beispielsweise die Alkali-, Erdalkali-, Ammonium-, Triethylamin- oder Tris-(2-hydroxyethyl)aminsalze sowie solche, die sich aus der Umsetzung von Verbindungen gemäß der Formel (IVa) oder (IVb) mit anorganischen und organischen Säuren wie Salzsäure, Phosphorsäure, Schwefelsäure, verzweigten oder unverzweigten, substituierten oder unsubstituierten (beispielsweise durch eine oder mehrere Hydroxygruppen) C₁ - C₄- Mono- oder Dicarbonsäuren, aromatische Carbonsäuren und Sulfonsäuren wie Essigsäure, Citronensäure, Benzoesäure, Maleinsäure, Fumarsäure, Weinsäure und p-Toluolsulfonsäure ergeben. Beispiele für besonders bevorzugte physiologisch verträgliche Salze sind die Na-, K-, Mg- und Ca- und Ammoniumsalze der Verbindungen gemäß der Formel (IVa) oder (IVb), sowie die Salze, die sich durch Umsetzung von Verbindungen gemäß der Formel (IVa) oder (IVb) mit Salzsäure, Essigsäure, Citronensäure und Benzoesäure ergeben.

Unter isomeren oder stereoisomeren Formen der Verbindungen gemäß Formel (IVa) oder (IVb) werden erfindungsgemäß alle auftretenden optischen Isomere, Diastereomere, Racemate, Zwitterionen, Kationen oder Gemische davon verstanden.

Unter dem Begriff Aminosäure werden die stereoisomeren Formen, z.B. D- und L-Formen, folgender Verbindungen verstanden:
Asparagin, Arginin, Asparaginsäure, Glutamin, Glutaminsäure, β-Alanin, γ-Aminobutyrat, N_{ε}-Acetyllysin, N_{δ}-Acetylornitin, Nγ-Acetyldiaminobutyrat, N_{α}-Acetyldiaminobutyrat, Histidin, Isoleucin, Leucin, Methionin, Phenylalanin, Serin, Threonin und Tyrosin.
L-Aminosäuren sind bevorzugt. Aminosäurereste leiten sich von den entsprechenden Aminosäuren ab. Die folgenden Aminosäurereste sind bevorzugt:
Gly, Ala, Ser, Thr, Val, β-Ala, γ-Aminobutyrat, Asp, Glu, Asn, Aln, N_{ε}-Acetyllysin, N_{δ}-Acetylornithin, N_{γ}-Acetyldiaminobutyrat, N_{α}-Acetyldiaminobutyrat.

Die Kurzschreibweise der Aminosäuren erfolgte nach der allgemein üblichen Schreibweise. Die Di- oder Tripeptidreste sind in ihrer chemischen Natur nach Säureamide und zerfallen bei der Hydrolyse in 2 oder 3 Aminosäuren. Die Aminosäuren in dem Di- oder Tripeptidrest sind durch Amidbindungen miteinander verbunden.

Bezüglich der Herstellung der Di- und Tripeptidreste wird ausdrücklich auf die EP 0 671 161 A1 der Firma Marbert verwiesen. Auch Beispiele für Di- und Tripeptidreste sind der Offenbarung der EP 0 671 161 A1 zu entnehmen.

Beispiele für C₁ - C₄-Alkylgruppen in den erfindungsgemäßen Verbindungen sind Methyl, Ethyl, n-Propyl, Isopropyl, n-Butyl, Isobutyl und tert.-Butyl. Bevorzugte Alkylgruppen sind Methyl und Ethyl, Methyl ist eine besonders bevorzugte Alkylgruppe. Bevorzugte C₂ - C₄-Hydroxyalkylgruppen sind die Gruppen 2-Hydroxyethyl, 3-Hydroxypropyl oder 4-Hydroxybutyl; 2-Hydroxyethyl ist eine besonders bevorzugte Hydroxyalkylgruppe.

Die erfindungsgemäßen Zweikomponentenmittel enthalten diese Wirkstoffe bevorzugt in Mengen von 0,001 bis 2, insbesondere von 0,01 bis 0,5 Gew.-%, jeweils bezogen auf die gesamte Anwendungszubereitung in dem jeweiligen Behälter.

Eine weitere erfindungsgemäß bevorzugte Gruppe von Pflegestoffen sind die Mono- bzw. Oligosaccharid.

Es können sowohl Monosaccharide als auch Oligosaccharide, wie beispielweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.

Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.

Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).

Die Mono- bzw. Oligosaccharide sind in den erfindungsgemäßen Haarbehandlungsmitteln bevorzugt in einer Menge von 0,1 bis 8 Gew.-%, insbesondere 1 bis 5 Gew.-%, bezogen auf die gesamte Anwendungszubereitung in dem jeweiligen Behälter, enthalten.

Weiterhin können als erfindungsgemäß bevorzugte Pflegestoffe die Silikonöle und/oder die Silikongums genannt werden.

Erfindungsgemäß geeignete Silikone oder Silikongums sind insbesondere Dialkyl- und Alkylarylsiloxane, wie beispielsweise Dimethylpolysiloxan und Methylphenyl-polysiloxan, sowie deren alkoxylierte, quaternierte oder auch anionische Derivate.

Beispiele für solche Silikone sind:
- Oligomere Polydimethylcyclosiloxane (INCI-Bezeichnung: Cyclomethicone), insbesondere die tetramere und die pentamere Verbindung, die als Handelsprodukte DC 344 bzw. DC 345 von Dow Corning vertrieben werden,
- Hexamethyl-Disiloxan (INCI-Bezeichnung: Hexamethyldisiloxane), z. B. das unter der Bezeichnung Abil^{®} K 520 vertriebenen Produkt,
- Polymere Polydimethylsiloxane (INCI-Bezeichnung: Dimethicone), z. B. die unter der Bezeichnung DC 200 von Dow Corning vertriebenen Produkte,
- Polyphenylmethylsiloxane (INCI-Bezeichnung: Phenyl Trimethicone), z. B. das Handelsprodukt DC 556 Fluid von Dow Corning,
- Silicon-Glykol-Copolymere (INCI-Bezeichnung: Dimethicone Copolyol), z. B. die Handelsprodukte DC 190 und DC 193 von Dow Corning,
- Ester sowie Partialester der Silicon-Glykol-Copolymere, wie sie beispielsweise von der Firma Fanning unter der Handelsbezeichnung Fancorsil^{®} LIM (INCI-Bezeichnung: Dimethicone Copolyol Meadowfoamate) vertrieben werden,
- Dimethylsiloxane mit Hydroxy-Endgruppen (INCI-Bezeichnung: Dimethiconol), z. B. die Handelsprodukte DC 1401 und Q2-1403 von Dow Corning,
- aminofunktionelle Polydimethylsiloxane und hydroxylaminomodifizierte Silicone (INCI-Bezeichnung: u. a. Amodimethicone und Quaternium-80), wie die Handelsprodukte XF42-B1989 (Hersteller GE Toshiba Silicones) Q2-7224 (Hersteller: Dow Corning; ein stabilisiertes Trimethylsilylamodimethicon), Dow Corning^{®} 939 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt),
- anionische Silikonöle, wie beispielsweise das Produkt Dow Corning^{®}1784
- aminomodifizierte Organosilicone, wie beispielsweise das Produkt Abil Soft A843 (Hersteller Osi Specialities).

Gemäß einer bevorzugten Ausführungsform enthalten die erfindungsgemäßen Zubereitungen eine Kombination aus einem flüchtigen und einem nichtflüchtigen Silikon. Flüchtig im Sinne der Erfindung sind solche Silikone, die ein Flüchtigkeit aufweisen, die gleich oder größer als die Flüchtigkeit des cyclischen, pentameren Dimethylsiloxans ist. Solche Kombinationen sind auch als Handelsprodukte (z. B. Dow Corning^{®}1401, Dow Corning^{®}1403 und Dow Corning^{®}1501, jeweils Mischungen aus einem Cyclomethicone und einem Dimethiconol) erhältlich.

Gemäß einer besonders bevorzugten Ausführungsform wird als Komponente (A) ein Dialkylpolysiloxan oder eines seiner Derivate eingesetzt. Bevorzugt sind die Alkylgruppen Methyl, Ethyl, i-Propyl und n-Propyl. Dimethylpolysiloxan oder eines seiner Derivate wird besonders bevorzugt eingesetzt. Bevorzugte sind die Derivate des Dimethylplysiloxans, die aminofunktionell sind. Ein ganz besonders bevorzugtes Derivat ist unter der INCI-Bezeichnung Amodimethicone im Handel erhältlich.

Die erfindungsgemäßen Zubereitungen enthalten die Silikone bevorzugt in Mengen von 0,01 - 10 Gew.-%, insbesondere 0,1 - 5 Gew.-%, bezogen auf die gesamte Anwendungszubereitung in dem jeweiligen Behälter.

Weiterhin kommen Lipide als erfindungsgemäß bevorzugte Pflegestoffe in Frage.

Erfindungsgemäß geeignete Lipide sind Phospholipide, beispielsweise Sojalecithin, EiLecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben.

Die erfindungsgemäßen Zubereitungen enthalten die Lipide bevorzugt in Mengen von 0,01 - 10 Gew.-%, insbesondere 0,1 - 5 Gew.-%, bezogen auf die gesamte Anwendungszubereitung in dem jeweiligen Behälter.

Ferner sind Ölkörper erfindungsgemäß bevorzugte Pflegestoffe.

Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyln-undecylether, n-Undecyl-n-dodecylether und n-Hexyl-n-Undecylether sowie Di-tert-butylether, Di-iso-pentylether, Di-3-ethyldecylether, tert.-Butyl-n-octylether, isoPentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykoldi-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-I),

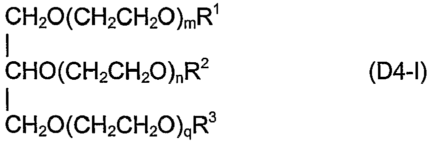

in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Zweikomponentenmitteln beträgt üblicherweise 0,1 - 30 Gew.%, bezogen auf die gesamte Anwendungszubereitung in dem jeweiligen Behälter, bevorzugt 0,1 - 20 Gew.-%, und insbesondere 0,1 - 15 Gew.-%.

Als weiterer erfindungsgemäß bevorzugter Pflegestoff kann ein Enzym eingesetzt werden. Erfindungsgemäß besonders bevorzugte Enzyme sind ausgewählt aus einer Gruppe, die gebildet wird aus Proteasen, Lipasen, Transglutaminase, Oxidasen und Peroxidasen.

Obwohl jeder der genannten, erfindungsgemäß bevorzugten Pflegestoffe für sich alleine bereits ein zufriedenstellendes Resultat ergibt, sind im Rahmen der vorliegenden Erfindung auch alle Ausführungsformen umfasst, in denen mehrere Pflegestoffe auch aus verschiedenen Gruppen eingesetzt werden.

In vielen Fällen enthalten die haarfarbverändernden Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)_{X}. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxy-ethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Färbemittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein weiterer Gegenstand der vorliegenden Anmeldung ist ein Verfahren zur Strähnchenapplikation, bei dem mindestens zwei verschiedene haarfarbverändernde Mittel mittels eines erfindungsgemäßen Abgabesystem gleichzeitig auf die Fasern aufgebracht werden, dort einige Zeit belassen werden, und die Fasern anschließend gründlich gespült werden.

Dabei kann sowohl eine Anwendung auf trockenem als auch eine Anwendung auf feuchtem Haar erfindungsgemäß bevorzugt sein. Eine besonders bevorzugte Form des feuchten Haares ist das handtuchtrockene Haar. Eine Anwendung auf trockenem Haar ist erfindungsgemäß ganz besonders bevorzugt.

Weiterhin spielt es erfindungsgemäß eine untergeordnete Rolle, welchen Vorbehandlungen das zu behandelnde Haar bereits ausgesetzt war. Die erfindungsgemäßen haarfarbverändernden Mittel können auf natürlichem, coloriertem und dauergewellten Haar zum Einsatz kommen.

Im Rahmen eines besonders bevorzugten Anwendungsverfahren werden die Fasern vor der Anwendung der haarfarbverändernden Mittel in Form gelegt. Unter in "Form legen" wird erfindungsgemäß das Herstellen der üblichen Haarfrisur verstanden. Auf diese Weise kann eine gezielte Behandlung der Haare mit dem Abgabesystem für haarfarbverändernde Mittel am einfachsten erfolgen. Dabei spielt es erfindungsgemäß keine wesentliche Rolle, ob die Haare im nassen oder im trockenen Zustand in Form gelegt werden. Das In-Form-Bringen kann mit Hilfe von unterschiedlichen Techniken erfolgen, so können die Haare beispielsweise mit einem Kamm oder mit einer Bürste in herkömmlicher Weise frisiert werden. Weiterhin kann mit dem erfindungsgemäßen Abgabesystem die Färbung sowohl im Deckhaar als auch in den unteren Haarpartien vorgenommen werden.
Die Anwendungszeit ist abhängig von den gewünschten Effekten sowie der Natur der haarfarbverändernden Mittel. Erfindungsgemäß bevorzugt sind Einwirkzeiten zwischen 2 und 120 Minuten, eine Einwirkzeit von 20 bis 45 ist besonders bevorzugt.

### Beispiele

### A) Formulierungsbeispiele

In einer Kunststoffflasche, deren Innenraum durch eine mittige Trennwand geteilt ist, werden jeweils die folgenden Zubereitungen A1 und A2 vorgelegt. Unmittelbar vor der Anwendung wird zu der Zubereitung A1 die Zubereitung B1 sowie ggf. die Zubereitung C1 und zu der Zubereitung A2 die Zubereitung B2 sowie ggf. die Zubereitung C2 hinzugegeben. Die Kunststoffflasche wird mit einem zweiteiligen Strähnchenaufsatz verschlossen und gründlich geschüttelt. Anschließend wird mit der Behandlung der Haare vom Kopfansatz bis hin zu den Spitzen begonnen. Nach einer Einwirkzeit von 30 Minuten bei Raumtemperatur werden die Haare gründlich gespült.

Die eingesetzten Zubereitungen werden im Folgenden durch die enthaltenden Bestandteile in INCI-Nomenklatur näher charakterisiert. Die Bestandteile sind dabei in abnehmender Reihenfolge im Hinblick auf die eingesetzte Menge angeordnet.

### 1 Beispiel 1

### 1.1 Vorgelegte Zubereitungen

### 1.1.1 Zubereitung A1

Aqua, Hydrogen Peroxide, Acrylates Copolymer, Etidronic Acid, Sodium Laureth Sulfate, 2,6-Dicarboxypyridine, Disodium Pyrophosphat, Dimethicone

### 1.1.2 Zubereitung A2

Aqua, Hydrogen Peroxide, Acrylates Copolymer, Etidronic Acid, Sodium Laureth Sulfate, 2,6-Dicarboxypyridine, Disodium Pyrophosphat, Dimethicone

### 1.2 Zuzugebende Zubereitungen

### 1.2.1 Zubereitung B1

Aqua, Cetearyl Alcohol, Sodium Laureth Sulfate, Cocomut Alcohol, Ammonium Hydroxide, Ammonium Sulfate, Etidronic Acid, Hydrolized Wheat Proteine, Sodium Silicate, Parfum, Linalool, Coumarin, Geraniol, Citronellol, Benzyl Benzoate

### 1.2.2 Zubereitung C1

Ammonium Persulfate, Silica

### 1.2.3 Zubereitung B2

Aqua, Cetearyl Alcohol, Sodium Laureth-6 Carboxylate, Coconut Alcohol, Sodium Myreth Sulfate, Ammonium Hydroxide, Octyldodecanol, Coco-Glucoside, Acrylamidopropyltrimmonium Chloride/Acrylates Copolymer, Ammonium Sulfate, Ceteareth-12, Ceteareth-20, Glyceryl Oleate, Sodium Sulfite, Ascorbic Acid, Etidronic Acid, Parfum, Toluene-2,5-Diamine Sulfate, 4-Amino-m-Cresol, m-Aminophenol, Resorcinol, 2-Methylresorcinol

### 1.2.4 Zubereitung C2

Aqua, Hydrolized Silk, Sericin, Hydroxypropylmethylcellulose, Glycol Stearate, Cocamide MEA, Laureth-10, Acrylamidopropyltrimmonium Chloride/Acrylates Copolymer, Sodium Sulfite, Ascorbic Acid, Sodium Benzoate, Sodium Salicylate, C.I.12200, C.I. 47005

### 2 Beispiel 2

### 2.3 Vorgelegte Zubereitungen

### 2.3.1 Zubereitung A1

Aqua, Hydrogen Peroxide, Acrylates Copolymer, Etidronic Acid, Sodium Laureth Sulfate, 2,6-Dicarboxypyridine, Disodium Pyrophosphat, Dimethicone

### 2.3.2 Zubereitung A2

Aqua, Hydrogen Peroxide, Acrylates Copolymer, Etidronic Acid, Sodium Laureth Sulfate, 2,6-Dicarboxypyridine, Disodium Pyrophosphat, Dimethicone

### 2.4 Zuzugebende Zubereitungen

### 2.4.1 Zubereitung B1

Aqua, Cetearyl Alcohol, Sodium Laureth Sulfate, Cocomut Alcohol, Ammonium Hydroxide, Ammonium Sulfate, Etidronic Acid, Hydrolized Wheat Proteine, Sodium Silicate, Parfum, Linalool, Coumarin, Geraniol, Citronellol, Benzyl Benzoate

### 2.4.2 Zubereitung C1

Ammonium Persulfate, Silica

### 2.4.3 Zubereitung B2

Aqua, Cetearyl Alcohol, Sodium Laureth-6 Carboxylate, Coconut Alcohol, Sodium Myreth Sulfate, Ammonium Hydroxide, Octyldodecanol, Coco-Glucoside, Acrylamidopropyltrimmonium Chloride/Acrylates Copolymer, Ammonium Sulfate, Ceteareth-12, Ceteareth-20, Glyceryl Oleate, Sodium Sulfite, Ascorbic Acid, Etidronic Acid, Parfum, Toluene-2,5-Diamine Sulfate, Resorcinol, 2-Methylresorcinol, o-Aminophenol

### 2.4.4 Zubereitung C2

Aqua, Hydrolized Silk, Sericin, Hydroxypropylmethylcellulose, Glycol Stearate, Cocamide MEA, Laureth-10, Acrylamidopropyltrimmonium Chloride/Acrylates Copolymer, Sodium Sulfite, Ascorbic Acid, Sodium Benzoate, Sodium Salicylate, C.I.12200, C.I. 47005

### 3 Beispiel 3

### 3.5 Vorgelegte Zubereitungen

### 3.5.1 Zubereitung A1

Aqua, Hydrogen Peroxide, Acrylates Copolymer, Etidronic Acid, Sodium Laureth Sulfate, 2,6-Dicarboxypyridine, Disodium Pyrophosphat, Dimethicone

### 3.5.2 Zubereitung A2

Aqua, Hydrogen Peroxide, Acrylates Copolymer, Etidronic Acid, Sodium Laureth Sulfate, 2,6-Dicarboxypyridine, Disodium Pyrophosphat, Dimethicone

### 3.6 Zuzugebende Zubereitungen

### 3.6.1 Zubereitung B1

Aqua, Cetearyl Alcohol, Sodium Laureth Sulfate, Coconut Alcohol, Ammonium Hydroxide, Ammonium Sulfate, Etidronic Acid, Hydrolized Wheat Proteine, Sodium Silicate, Parfum, Linalool, Coumarin, Geraniol, Citronellol, Benzyl Benzoate

### 3.6.2 Zubereitung C1

entfällt

### 3.6.3 Zubereitung B2

Aqua, Cetearyl Alcohol, Sodium Laureth-6 Carboxylate, Coconut Alcohol, Sodium Myreth Sulfate, Ammonium Hydroxide, Octyldodecanol, Coco-Glucoside, Acrylamidopropyltrimmonium Chloride/Acrylates Copolymer, Ammonium Sulfate, Ceteareth-12, Ceteareth-20, Glyceryl Oleate, Sodium Sulfite, Ascorbic Acid, Etidronic Acid, Parfum, Toluene-2,5-Diamine Sulfate, 4-Amino-m-Cresol, m-Aminophenol, Resorcinol, 2-Methylresorcinol, 4-Chlorresorcinol

### 3.6.4 Zubereitung C2

Aqua, Hydrolized Silk, Sericin, Hydroxypropylmethylcellulose, Glycol Stearate, Cocamide MEA, Laureth-10, Acrylamidopropyltrimmonium Chloride/Acrylates Copolymer, Sodium Sulfite, Ascorbic Acid, Sodium Benzoate, Sodium Salicylate, C.I.12200, C.I. 47005

### 4 Beispiel 4

### 4.7 Vorgelegte Zubereitungen

### 4.7.1 Zubereitung A1

Aqua, Hydrogen Peroxide, Acrylates Copolymer, Etidronic Acid, Sodium Laureth Sulfate, 2,6-Dicarboxypyridine, Disodium Pyrophosphat, Dimethicone

### 4.7.2 Zubereitung A2

Aqua, Hydrogen Peroxide, Acrylates Copolymer, Etidronic Acid, Sodium Laureth Sulfate, 2,6-Dicarboxypyridine, Disodium Pyrophosphat, Dimethicone

### 4.8 Zuzugebende Zubereitungen

### 4.8.1 Zubereitung B1

Aqua, Cetearyl Alcohol, Ammonium Hydroxide, Coconut Alcohol, Isostearic Acid, Sodium Laureth Sulfate, Cocamidopropylbetaine, Potassium Hydroxide, Myristic Acid, Ceteareth-12, Ceteareth-20, Sodium Sulfite, Ammonium Sulfate, Ascorbic Acid, Sodium Silicate, Carbomer, Etidronic Acid, Propylene Glykol, Parfum, Linalool, Citronellol, Toluene-2,5-Diamine Sulfate, 4-Amino-2-Hydroxytoluene, Resorcinol, 2-Methylresorcinol, 4-Chlorresorcinol, 2-Amino-4-Hydroxyethylaminoanisole Sulfate

### 4.8.2 Zubereitung C1

entfällt

### 4.8.3 Zubereitung B2

Aqua, Cetearyl Alcohol, Sodium Laureth-6 Carboxylate, Coconut Alcohol, Sodium Myreth Sulfate, Ammonium Hydroxide, Octyldodecanol, Coco-Glucoside, Acrylamidopropyltrimmonium Chloride/Acrylates Copolymer, Ammonium Sulfate, Ceteareth-12, Ceteareth-20, Glyceryl Oleate, Sodium Sulfite, Ascorbic Acid, Etidronic Acid, Parfum, Toluene-2,5-Diamine Sulfate, Resorcinol, 2-Methylresorcinol, 2-Amino-3-hydroxypyridine, Hydroxyethyl-2-Nitro-P-Toluidine,

### 4.8.4 Zubereitung C2

entfällt

### B) Ausführungsbeispiel für ein erfindungsgemäßes Abgabesystem

Im Folgenden werden weitere Merkmale, Ziele und Vorteile der Erfindung anhand eines bevorzugten Ausführungsbeispiels näher erläutert. In der Zeichnung zeigt
Fig. 1 ein erfindungsgemäßes Abgabesystem für haarfarbverändernde Mittel,
Fig. 2 einen Behälter aus Fig. 1 in Seitenansicht.

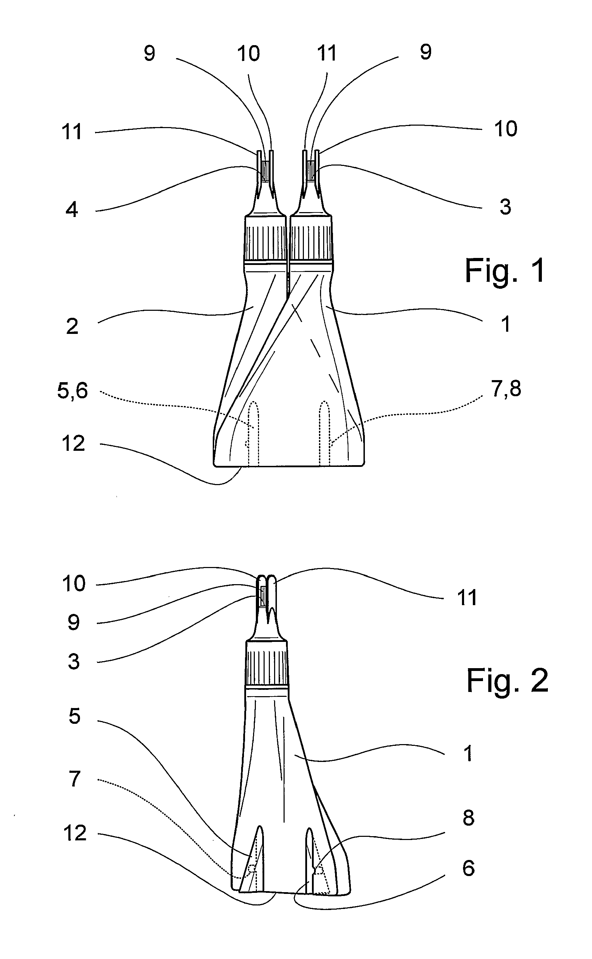

Das hier gezeigte Abgabesystem für haarfarbverändernde Mittel (Fig. 1) umfasst hier und vorzugsweise genau zwei Behälter 1, 2. Die Behälter 1, 2 weisen jeweils eine Austrittsöffnung 3, 4 zur Abgabe eines haarfarbverändernen Mittels auf. Anstelle nur einer Austrittsöffnung 3, 4 kann jeder Behälter 1, 2 auch mehrere Austrittsöffnungen aufweisen. Ebenso können auch mehr als zwei Behälter 1, 2 für haarfarbverändernde Mittel vorgesehen seien. Die Austrittsöffnungen 3, 4 sind räumlich voneinander isoliert angeordnet, so dass eine Vermischung der haarfarbverändernden Mittel im Wesentlichen nicht stattfindet. Erfindungsgemäß ist nun vorgesehen, dass die Behälter 1, 2 unterschiedliche haarfarbverändernde Mittel enthalten. Dadurch ist es möglich, gleichzeitig unterschiedliche haarfarbverändernde Mittel an voneinander beabstandeten Orten aufzutragen. Dies führt zu einer erheblichen Zeitersparnis, beispielsweise beim Färben von Strähnen.

Hier und vorzugsweise ist vorgesehen, dass einer der Behälter 1, 2 ein Blondiermittel enthält und der andere Behälter 1, 2 ein oxidatives Färbemittel. Es können allerdings auch andere haarfarbverändernde Mittel in den Behältern 1, 2 vorgesehen seien. Hierzu und in Bezug auf die weitere Ausgestaltung der Produktchemie darf auf die vorherige Beschreibung verwiesen werden.

Der allgemeinen Beschreibung ist zu entnehmen, dass das Abgabesystem einstückig, also aus einem Guss, gestaltet sein kann. Erfindungsgemäß ist jedoch vorgesehen, dass die Behälter 1, 2 voneinander getrennt ausgeführt und aneinander ansteckbar, also lösbar koppelbar, sind. Die lösbare Kopplung ermöglicht dann die Anwendung auch nur eines der haarfarbverändernden Mittel aus einem der Behälter 1, 2.

Die lösbare Kopplung der Behälter 1, 2 erfolgt hier und vorzugsweise mittels einer Feder-Nut-Anordnung (Fig. 2). Hierzu weist der eine Behälter 1 an seiner Oberfläche eine Feder 5 und der andere Behälter 2 an seiner Oberseite eine der Feder 5 des ersten Behälters 1 zugeordnete Nut 6 auf. Hier und vorzugsweise weisen beiden Behälter 1, 2 sowohl eine Feder 5 als auch eine Nut 6 auf. Diese wirken jeweils mit Feder 5 und Nut 6 des anderen Behälters 1, 2 zusammen. Zusätzlich oder alternativ dazu können auch andere Rastvorrichtungen vorgesehen sein.

Hier und vorzugsweise ist ferner vorgesehen, dass die Nut 6 eine Rastnoppe 7 aufweist. In der Feder 5 ist entsprechend eine Rastvertiefung 8 vorgesehen, in der die Rastnoppe 7 bei gekoppelten Behältern 1, 2 einrastet. Die Rastnoppe 7 dient damit gleichzeitig als Sperre gegen ein vertikales Verrutschen der Behälter 1, 2 gegeneinander.

In Fig. 1 ist dargestellt, dass um die Austrittsöffnungen 3, 4 herum Borsten 9 angeordnet sind. Die Borsten 9 dienen dazu eine größere Oberfläche zur Verteilung des jeweiligen haarfarbverändernden Mittels im Haar bereitzustellen. Alternativ zu der Anordnung der Borsten 9 um die Austrittsöffnungen 3, 4 herum ist es auch möglich, die Borsten 9 so anzuordnen, dass sie praktisch innerhalb der Austrittsöffnungen 3, 4 angeordnet sind, dass heißt, dass das haarfarbverändernde Mittel beim Austritt aus dem Behälter 1, 2 an den Borsten 9 entlang fließt und mit diesen auf dem Haar aufgetragen wird.
Anstelle der Borsten 9 oder zusätzlich zu diesen kann nach den Austrittsöffnungen 3, 4 ein Schwamm angeordnet sein (nicht gezeigt). Mit "nach der Austrittsöffnung 5, 6" ist dabei eine Position gemeint, die in Fließrichtung des haarfarbverändernden Mittels hinter der Austrittsöffnung 3, 4 liegt. Auch ist es möglich, dass in mindestens eine der Austrittsöffnungen 3, 4 ein Faserverbund reinreicht. Der Schwamm und der Faserverbund dienen insofern ebenfalls dem Zweck einer gleichmäßigen Verteilung des haarfarbverändernden Mittels auf dem Haar.

Zu diesem Zweck kann es auch vorgesehen sein, dass nach mindestens einer der Austrittsöffnungen 3, 4 ein Kugelventil angeordnet ist (nicht gezeigt). Ein solches Kugelventil kann beispielsweise so ausgeführt sein, dass es die Austrittsöffnung 3, 4 an sich verschließt und nur in Gebrauchsstellung des Abgabesystems freigibt. Die Gebrauchsstellung des Abgabesystems ist vorliegend eine Stellung in der das Abgabesystem mit den Austrittsöffnungen 3, 4 nach unten gehalten wird. In der Gebrauchsstellung kann dann das haarfarbverändernde Mittel an der Kugel des Kugelventils vorbei und durch die Austrittsöffnungen 3, 4 strömen.

Eine weitere Möglichkeit zum Auftragen des haarfarbverändernden Mittels auf dem Haar ist die Anordnung eines von Watte umgebenen Stabs nach mindestens einer der Austrittsöffnungen 3, 4. Dieser von Watte umgebene Stab kann einerseits zum Auftragen des haarfarbverändernden Mittels verwendet werden, er kann aber auch vorgesehen sein, um die Auftragung des haarfarbverändernden Mittels zur Seite hin zu begrenzen und überschüssiges haarfarbveränderndes Mittel aufzunehmen.

Die zuvor beschriebenen Ausgestaltungen (Borsten, Schwamm, Faserverbund, Kugelventil, von Watte umgebener Stab) können an den Austrittsöffnungen 3, 4 miteinander kombiniert werden. Es können aber auch Unterkombinationen vorgesehen sein sowie unterschiedliche Kombinationen für die verschiedenen Austrittsöffnungen 3, 4. Die konkrete Ausgestaltung richtet sich dabei vorzugsweise nach dem haarfarbverändernden Mittel, insbesondere nämlich nach dessen Konsistenz. Ferner ist vorgesehen, dass die zwei Austrittsöffnungen 3, 4 durch Trennwände voneinander getrennt sind. Diese Trennwände sollen verhindern, dass eine Durchmischung der haarfarbverändernden Mittel in dem Haar stattfindet. Sie sind somit eine Ergänzung zu der räumlich isolierten Anordnung der Austrittsöffnungen 3, 4.

In Fig. 1 ist dargestellt, dass an dem Rand der Austrittsöffnungen 3, 4 von dort in Fließrichtung abstehende Vorsprünge 10, 11 vorgesehen sind. Diese Vorsprünge 10, 11 sind jeweils auf einander gegenüberliegenden Seiten der Austrittsöffnungen 3, 4 angeordnet. Die Vorsprünge 10, 11 dienen damit zum Schutz des umliegenden Haars vor dem haarfarbverändernden Mittel. Gleichzeitig nehmen die Vorsprünge 10, 11 eine Führungsfunktion zum Auftragen des haarfarbverändernden Mittels wahr. Die Vorsprünge 10, 11 sind nämlich an nur zwei Seiten der Austrittsöffnung 3, 4 vorgesehen und lassen die weiteren Seiten frei. Dies ermöglicht eine einfache Führung des Abgabesystems entlang einzelner Haarsträhnen.

Aus der Zeichnung ist ferner ersichtlich, dass die Behälter 1, 2 identisch ausgeführt sind. Dies ist insbesondere im Hinblick auf die Fertigung besonders bevorzugt, da hierdurch nur ein Werkzeug zur Herstellung der Behälter 1, 2 erforderlich ist. Für die identische Ausführung der Behälter 1, 2 ist die Feder-Nut-Anordnung besonders geeignet, da sich die Zuordnung von Feder 5 und Nut 6 der jeweiligen Behälter 1, 2 durch die Fertigung praktisch von selbst ergibt.

Den Figuren 1, 2 ist zu entnehmen, dass jeder Behälter 1, 2 eine im wesentlichen ovale Grundfläche mit einer abgeflachten längeren Außenseite 12 aufweist. Die Behälter 1, 2 sind nun derart angeordnet, dass die jeweils abgeflachten längeren Außenseiten 12 zueinander hinzeigen. Diese Anordnung ist zum Halten des Abgabesystems besonders vorteilhaft. Ferner eignet sich diese Ausgestaltung in besonderer Weise dann, wenn die Ausbringung der haarfarbverändernden Mittel durch Druckausübung auf die Wände der Behälter 1, 2 erfolgt. Die Druckausübung erfolgt bei dieser Ausgestaltung nämlich auf die längeren Außenseiten der Behälter 1, 2 im Wesentlichen auf gleicher Höhe und mit der gleichen Kraft. Dies ermöglicht eine relativ gleichmäßige Ausbringung der beiden haarfarbverändernden Mittel. Auch hierfür ist die identische Ausführung der Behälter 1, 2 von besonderem Vorteil.
Hier sind die Vorsprünge 10, 11 im Wesentlichen in einer Ebene parallel zu den kürzeren Außenseiten der Behälter 1, 2 angeordnet. Eine solche Anordnung ist für die Handhabung des Abgabesystems optimal. Dieses kann von den kürzeren Außenseiten her gut gegriffen werden. Ferner ermöglichen die Vorsprünge 10, 11 als Führung eine vertikale Auf- bzw. Abbewegung des Abgabesystems, so dass dieses bei der Verwendung nicht unnötigerweise hin- und hergedreht werden muss.

### C) Weitere Formulierungen

Im Rahmen dieser Formulierungen wird in den Kammern des unter Punkt B) beschriebenen Abgabesystems jeweils die Oxidationsmittelzubereitung, die unter Punkt C) 6 beschrieben wird, vorgelegt. Unmittelbar vor der Anwendung wird dann in jeder Kammer des Abgabesystems ein farbveränderendes Mittel, wie unter den Punkten C)1 bis C)5 beschrieben, hinzugefügt. Nach kräftigem Schütteln werden die resultierenden Anwendungszubereitungen auf die Fasern aufgetragen und nach einer Einwirkzeit wieder abgespült.

### 1 Blondiermittel

| | |
|---|---|
| Hydrenol^{®} D | 9,0 |
| Lorol^{®} techn. | 3,0 |
| Texapon^{®} NSO | 7,0 |
| Plantapon^{®} LGC | 5,0 |
| Eumulgin^{®} B 1 | 0,5 |
| Eumulgin^{®} B 2 | 0,5 |
| Prisorine^{®} 3501 | 2,0 |
| Edenor^{®} C14 | 0,5 |
| Kaliumhydroxid 50% | 1,2 |
| Natriumsulfit | 0,5 |
| Ascorbinsäure | 0,4 |
| Ammoniumsulfat | 0,4 |
| Ammoniak 25 % | 0,4 |
| Natriumsilikat 40/42 | 0,5 |
| Turpinal^{®} SL | 0,2 |
| Parfum | 0,2 |
| Ammoniak 25 % | 5,7 |
| Wasser | ad 100 |

### 2 Färbmittel der Nuance Caramel I

| | |
|---|---|
| Hydrenol^{®} D | 9,0 |
| Lorol^{®} techn. | 3,0 |
| Texapon^{®} NSO | 7,0 |
| Plantapon^{®} LGC | 5,0 |
| Eumulgin^{®} B 1 | 0,5 |
| Eumulgin^{®} B 2 | 0,5 |
| Prisorine^{®} 350 | 12,0 |
| Edenor^{®} C14 | 0,5 |
| Kaliumhydroxid 50% | 1,2 |
| m-Aminophenol | 0,01 |
| p-Toluylendiaminsulfat | 0,01 |
| 2-Amino-3-hydroxypyridin | 0,002 |
| Hydroxyethyl-2-nitro-p-toluidin | 0,05 |
| Resorcin | 0,04 |
| 2-Methylresorcin | 0,01 |
| Natriumsulfit | 0,5 |
| Ascorbinsäure | 0,4 |
| Ammoniumsulfat | 0,5 |
| Ammoniak 25 % | 0,15 |
| Natriumsilikat 40/42 | 0,5 |
| Turpinal^{®} SL | 0,2 |
| Parfum | 0,4 |
| Ammoniak 25 % | 5,3 |
| Wasser | ad 100 |

### 3 Färbmittel der Nuance Caramel II

| | |
|---|---|
| Hydrenol^{®} D | 9,0 |
| Lorol^{®} techn. | 3,0 |
| Texapon^{®} NSO | 7,0 |
| Plantapon^{®} LGC | 5,0 |
| Eumulgin^{®} B 1 | 0,5 |
| Eumulgin^{®} B 2 | 0,5 |
| Prisorine^{®} 3501 | 2,0 |
| Edenor^{®} C14 | 0,5 |
| Wasser, vollentsalzt | 12,0 |
| Kaliumhydroxid 50% | 1,2 |
| Natriumsulfit | 0,5 |
| Ascorbinsäure | 0,4 |
| Ammoniumsulfat | 0,4 |
| p-Toluylendiaminsulfat | 0,42 |
| Resorcin | 0,2 |
| m-Aminophenol | 0,01 |
| Ammoniak 25 % | 0,4 |
| 5-Amino-2-methylphenol | 0,01 |
| 1,2-Propandiol | 0,02 |
| 4-Amino-3-nitrophenol | 0,06 |
| Natriumsilikat 40/42 | 0,5 |
| Turpinal^{®} SL | 0,2 |
| Parfum | 0,2 |
| Ammoniak 25 % | 5,7 |
| Wasser | ad 100 |

### 4 Färbmittel der Nuance Mahagoni

| | |
|---|---|
| Hydrenol^{®} D | 9,0 |
| Lorol^{®} techn. | 3,0 |
| Texapon^{®} NSO | 7,0 |
| Plantapon^{®} LGC | 5,0 |
| Eumulgin^{®} B 1 | 0,5 |
| Eumulgin^{®} B 2 | 0,5 |
| Prisorine^{®} 3501 | 2,0 |
| Edenor^{®} C14 | 0,5 |
| Kaliumhydroxid 50% | 1,2 |
| 5-Amino-2-methylphenol | 0,56 |
| 1,2-Propandiol | 1,6 |
| 2,7-Dihydroxynaphthalin | 0,09 |
| p-Toluylendiaminsulfat | 0,6 |
| Resorcin | 0,14 |
| Bis-(5-Amino-2-hydroxyphenyl)methan 2HCl | 1,02 |
| Natriumsulfit | 0,5 |
| Ascorbinsäure | 0,4 |
| Ammoniumsulfat | 0,1 |
| Ammoniak 25 % | 1,2 |
| Natriumsilikat 40/42 | 0,5 |
| Turpinal^{®} SL | 0,2 |
| Parfum | 0,4 |
| Ammoniak 25 % | 5,8 |
| Wasser | ad 100 |

### 5 Färbmittel der Nuance Mocca

| | |
|---|---|
| Hydrenol^{®} D | 9,0 |
| Lorol^{®} techn. | 3,0 |
| Texapon^{®} NSO | 7,0 |
| Plantapon^{®} LGC | 5,0 |
| Eumulgin^{®} B 1 | 0,5 |
| Eumulgin^{®} B 2 | 0,5 |
| Prisorine^{®} 3501 | 2,0 |
| Edenor^{®} C14 98/100 | 0,5 |
| Kaliumhydroxid 50% | 1,2 |
| Resorcin | 0,55 |
| p-Toluylendiaminsulfat | 1,43 |
| m-Aminophenol | 0,15 |
| 3-Amino-2-methylamino-6-methoxypyridin | 0,04 |
| Natriumsulfit wasserfrei A 96% MIN | 0,5 |
| Ascorbinsäure E 300 DAB | 0,4 |
| Ammoniak 25 % | 1,8 |
| Natriumsilikat 40/42 | 0,5 |
| Turpinal^{®} SL | 0,2 |
| Parfum | 0,4 |
| Ammoniak 25 % | 4,0 |
| Wasser | ad 100 |

### 6 Eingesetzte Oxidationsmittelzubereitung

| | |
|---|---|
| Ammoniak 25 % | 0,62 |
| Dipicolinsäure | 0,1 |
| Dinatriumpyrophosphat | 0,03 |
| Turpinal^{®} SL | 1,5 |
| Texapon^{®} NSO | 2,0 |
| Silikonemulsion 10% | 0,07 |
| Acrysol^{®} 33 | 12,0 |
| Wasserstoffperoxid 50 % | 22,4 |
| Wasser | ad 100 |

### 7 Verzeichnis der eingesetzten Handelsprodukte

| | |
|---|---|
| Acrysol^{®} 33 | Acrylpolymer (ca. 28% Festkörper in Wasser; INCI-Bezeichnung: Acrylates Copolymer) |
| Edenor^{®} C14 | Myristinsäure (INCI-Bezeichnung: MYRISTIC ACID) (Cognis) |
| Eumulgin^{®} B 1 | Cetylstearylalkohol mit ca. 12-EO-Einheiten (INCI-Bezeichnung: Ceteareth-12) (Cognis) |
| Eumulgin^{®} B2 | Cetylstearylalkohol mit ca. 20 EO-Einheiten (INCI-Bezeichnung: Ceteareth-20) (Cognis) |
| Hydrenol^{®} D | C₁₆₋₁₈-Fettalkohol (INCI-Bezeichnung: Cetearyl alcohol) (Cognis) |
| Lorol^{®} tech. | C₁₂₋₁₈-Fettalkohol (INCI-Bezeichnung: Coconut alcohol) (Cognis) |
| Plantapon^{®} LGC | ca. 28-34% Aktivsubstanzgehalt in Wasser; INCI-Bezeichnung: Lauryl Glucose Carboxylate, Lauryl Glucoside (Cognis) |
| Prisorine^{®} 3501 | Isooctadecansäure (INCI-Bezeichnung: Isostearic Acid) (Uniqema) |
| Texapon^{®} NSO | Laurylethersulfat, Natriumsalz (ca. 27,5% Aktivsubstanz; INCI-Bezeichnung: Sodium Laureth Sulfate) (Cognis) |
| Turpinal^{®} SL | 1-Hydroxyethan-1,1-diphosphonsäure (ca. 58 - 61% Aktivsubstanzgehalt; INCI-Bezeichnung: Etidronic Acid, Aqua (Water)) (Solutia) |

## Patentansprüche

1. Abgabesystem für haarfarbverändernde Mittel, umfassend mindestens zwei lösbar koppelbare Behälter (1, 2), die unterschiedliche haarfarbverändernde Mittel enthalten, wobei jeder dieser Behälter (1, 2) mindestens eine Austrittsöffnung (3, 4) aufweist, wobei die Behälter (1, 2) jeweils abgeflachte längere Außenseiten (12) aufweisen und mit diesen abgeflachten längeren Außenseiten (12) zueinander angeordnet sind, **dadurch gekennzeichnet,**
- **dass** die Austrittsöffnungen (3, 4) voneinander räumlich so isoliert angeordnet sind, dass die unterschiedlichen haarfarbverändernden Mittel gleichzeitig an voneinander beabstandeten Orten austragbar sind,
- **dass** der Rand einer Austrittsöffnung (3, 4) zwei, auf gegenüberliegenden Seiten der Austrittsöffnung (3, 4) angeordnete und in Fließrichtung abstehende Vorsprünge (10, 11) aufweist, welche im Wesentlichen in einer Ebene parallel zu den kürzeren Außenseiten der Behälter (1, 2) angeordnet sind, wobei zwischen den Vorsprüngen (10, 11) Borsten (9) oder ein Auftragsschwämmchen zum gleichmäßigen Auftragen der Mittel angeordnet sind.

2. Abgabesystem nach Anspruch 1, **dadurch gekennzeichnet, dass** mindestens ein Behälter (1, 2) ein Blondiermittel enthält.

3. Abgabesystem nach einem der Ansprüche 1 oder 2, **dadurch gekennzeichnet, dass** mindestens ein Behälter (1, 2) ein oxidatives Färbemittel enthält.

4. Abgabesystem nach einem der Ansprüche 1 bis 3, **dadurch gekennzeichnet, dass** die Behälter (1, 2) geometrisch so geformt sind, dass sie aneinander ansteckbar sind.

5. Abgabesystem nach Anspruch 4, **dadurch gekennzeichnet, dass** ein Behälter (1, 2) an seine Oberfläche mindestens eine Nut (6) und der andere Behälter (1, 2) mindestens eine, darin eingreifende Feder (5) aufweist.

6. Abgabesystem nach einem der Ansprüche 1 bis 5, **dadurch gekennzeichnet, dass** die Behälter (1, 2) einander mindestens teilweise umgreifen.

7. Abgabesystem nach einem der Ansprüche 4 bis 6, **dadurch gekennzeichnet, dass** einer der Behälter (1, 2) an seiner Oberfläche mindestens eine Rastnoppe (7) und der andere Behälter (1, 2) eine dazu korrespondierende Rastvertiefung (8) aufweist.

8. Abgabesystem nach Anspruch 5 und ggf. nach Anspruch 6 oder 7, **dadurch gekennzeichnet, dass** die Nut (6) und die Feder (5) zusätzlich mindestens eine Rastnoppe (7) und eine dazu korrespondierende Rastvertiefung (8) aufweisen.

9. Abgabesystem nach einem der Ansprüche 1 bis 8, **dadurch gekennzeichnet, dass** um mindestens eine Austrittsöffnung (3, 4) Borsten (9) angeordnet sind.

10. Abgabesystem nach einem der Ansprüche 1 bis 9, **dadurch gekennzeichnet, dass** nach mindestens einer Austrittsöffnung (3, 4) ein Schwamm angeordnet ist.

11. Abgabesystem nach einem der Ansprüche 1 bis 10, **dadurch gekennzeichnet, dass** in mindestens eine Austrittsöffnung (3, 4) ein Faserverbund reinreicht.

12. Abgabesystem nach einem der Ansprüche 1 bis 11, **dadurch gekennzeichnet, dass** nach mindestens einer Austrittsöffnung (3, 4) ein Kugelventil angeordnet ist.

13. Abgabesystem nach einem der Ansprüche 1 bis 12, **dadurch gekennzeichnet, dass** nach mindestens einer Austrittsöffnung (3, 4) ein von Watte umgebender Stab angeordnet ist.

14. Abgabesystem nach einem der Ansprüche 1 bis 13, **dadurch gekennzeichnet, dass** nach mindestens einer Austrittsöffnung (3, 4) ein Kamm angeordnet ist.

15. Abgabesystem nach einem der Ansprüche 1 bis 14,
**dadurch gekennzeichnet, dass**
die Behälter (1, 2) identisch ausgeführt sind.

16. Abgabesystem nach einem der Ansprüche 1 bis 15,
**dadurch gekennzeichnet, dass**
mindestens eines der haarfarbverändernden Mittel mindestens einen Pflegestoff enthält.

17. Abgabesystem nach einem der Ansprüche 1 bis 16,
**dadurch gekennzeichnet, dass**
mindestens eines der haarfarbverändernden Mittel mindestens ein Tensid enthält.

18. Verfahren zur Strähnchenapplikation, **dadurch gekennzeichnet, dass** mindestens zwei verschiedene haarfarbverändernde Mittel mittels eines Abgabesystems gemäß einem der Ansprüche 1 bis 17 gleichzeitig auf die Fasern aufgebracht werden, dort einige Zeit belassen werden, und die Fasern anschließend gründlich gespült werden.

19. Verfahren nach Anspruch 18,
**dadurch gekennzeichnet, dass** die Fasern vor der Anwendung der haarfarbverändernden Mittel in Form gelegt werden.

## Claims

1. A delivery system for hair-colour-modifying agents, comprising at least two releasably couplable containers (1, 2) that contain different hair-colour-modifying agents, wherein each of said containers (1, 2) comprises at least one outlet opening (3, 4), wherein the containers (1, 2) each comprise flattened longer outer sides (12) and are arranged with said flattened longer outer sides (12) toward one another, **characterized in that**
- the outlet openings (3, 4) are arranged in a manner spatially isolated from one another so that the different hair-colour-modifying agents can be applied simultaneously at locations spaced apart from one another;
- the edge of one outlet opening (3, 4) comprises two projections (10, 11), arranged on oppositely located sides of the outlet opening (3, 4) and protruding in a flow direction, which are arranged substantially in a plane parallel to the shorter outer sides of the containers (1, 2), wherein bristles (9) or an applicator sponge for uniform application of the agents are arranged between the projections (10, 11).

2. The delivery system according to Claim 1, **characterized in that** at least one container (1, 2) contains a hair-bleaching agent.

3. The delivery system according to one of Claims 1 or 2, **characterized in that** at least one container (1, 2) contains an oxidizing colouring agent.

4. The delivery system according to one of Claims 1 to 3, **characterized in that** the containers (1, 2) are geometrically shaped so that they are mountable onto one another.

5. The delivery system according to Claim 4, **characterized in that** one container (1, 2) comprises on its surface at least one groove (6), and the other container (1, 2) at least one spring (5) engaging thereinto.

6. The delivery system according to one of Claims 1 to 5, **characterized in that** the containers (1, 2) at least partly fit around one another.

7. The delivery system according to one of Claims 4 to 6, **characterized in that** one of the containers (1, 2) comprises on its surface at least one snap-lock knob (7), and the other container (1, 2) a snap-lock depression (8) corresponding thereto.

8. The delivery system according to Claim 5 and optionally according to Claim 6 or 7, **characterized in that** the groove (6) and the spring (5) additionally comprise at least one snap-lock knob (7) and a snap-lock depression (8) corresponding thereto.

9. The delivery system according to one of Claims 1 to 8, **characterized in that** bristles (9) are arranged around at least one outlet opening (3, 4).

10. The delivery system according to one of Claims 1 to 9, **characterized in that** a sponge is arranged after at least one outlet opening (3, 4).

11. The delivery system according to one of Claims 1 to 10, **characterized in that** a fibre composite extends into at least one outlet opening (3, 4).

12. The delivery system according to one of Claims 1 to 11, **characterized in that** a ball valve is arranged after at least one outlet opening (3, 4).

13. The delivery system according to one of Claims 1 to 12, **characterized in that** a rod surrounded by batting is arranged after at least one outlet opening (3, 4).

14. The delivery system according to one of Claims 1 to 13, **characterized in that** a comb is arranged after at least one outlet opening (3, 4).

15. The delivery system according to one of Claims 1 to 14, **characterized in that** the containers (1, 2) are embodied identically.

16. The delivery system according to one of Claims 1 to 15, **characterized in that** at least one of the hair-colour-modifying agents contains at least one care-providing substance.

17. The delivery system according to one of Claims 1 to 16, **characterized in that** at least one of the hair-colour-modifying agents contains at least one surfactant.

18. A method for strand application, **characterized in that** at least two different hair-colour-modifying agents are simultaneously applied by means of a delivery system in accordance with one of Claims 1 to 17 onto the fibres, left there for some time, and then the fibres are thoroughly rinsed.

19. The method according to Claim 18, **characterized in that** the fibres are brought into a shape before utilization of the hair-colour-modifying agents.

## Revendications

1. Système de distribution pour des agents modifiant la couleur des cheveux, comprenant au moins deux récipients (1, 2) aptes à être accouplés de manière amovible, qui contiennent des agents différents modifiant la couleur des cheveux, chacun de ces récipients (1, 2) présentant au moins une ouverture de sortie (3, 4), les récipients (1, 2) présentant à chaque fois des grands côtés externes aplatis (12) et étant disposés l'un par rapport à l'autre avec ces grands côtés externes aplatis (12), **caractérisé en ce que** les ouvertures de sortie (3, 4) sont disposées en étant isolées dans l'espace l'une par rapport à l'autre de telle sorte que les agents différents modifiant la couleur des cheveux peuvent être appliqués de manière simultanée à des endroits situés à l'écart l'un de l'autre, **en ce que** le bord d'une ouverture de sortie (3, 4) présente deux protubérances (10, 11) disposées sur les côtés opposés de l'ouverture de sortie (3, 4) et faisant saillie dans la direction d'écoulement, qui sont disposées essentiellement dans un plan parallèlement aux petits côtés externes des récipients (1, 2), des brosses (9) ou une petite éponge d'application étant disposées entre les protubérances (10, 11) pour une application uniforme des agents.

2. Système de distribution selon la revendication 1, **caractérisé en ce qu'**au moins un récipient (1, 2) contient un agent de décoloration.

3. Système de distribution selon l'une quelconque des revendications 1 ou 2, **caractérisé en ce qu'**au moins un récipient (1, 2) contient une teinture oxydative.

4. Système de distribution selon l'une quelconque des revendications 1 à 3, **caractérisé en ce que** les récipients (1, 2) possèdent une configuration géométrique telle qu'ils peuvent venir se disposer l'un contre l'autre.

5. Système de distribution selon la revendication 4, **caractérisé en ce qu'**un récipient (1, 2) présente à sa surface au moins une rainure (6) et l'autre récipient (1, 2) présente au moins une plume (5) qui vient s'y insérer.

6. Système de distribution selon l'une quelconque des revendications 1 à 5, **caractérisé en ce que** les récipients (1, 2) s'entourent ['un l'autre au moins en partie.

7. Système de distribution selon l'une quelconque des revendications 4 à 6, **caractérisé en ce qu'**un récipient (1, 2) présente à sa surface au moins un bouton d'encliquetage (7) et l'autre récipient ('f , 2) présente un renfoncement d'encliquetage (8) y correspondant.

8. Système de distribution selon la revendication 5, et de manière facultative selon la revendication 6 ou 7, **caractérisé en ce que** la rainure (6) et la plume (5) présentent en outre au moins un bouton d'encliquetage (7) et un renfoncement d'encliquetage (8) y correspondant.

9. Système de distribution selon l'une quelconque des revendications 1 à 8, **caractérisé en ce que** des brosses (9) sont disposées autour d'au moins une ouverture de sortie (3, 4).

10. Système de distribution selon l'une quelconque des revendications 1 à 9, **caractérisé en ce qu'**une éponge est disposée après au moins une ouverture de sortie (3, 4).

11. Système de distribution selon l'une quelconque des revendications 1 à 10, **caractérisé en ce qu'**une fibre composite pénètre dans au moins une ouverture de sortie (3, 4).

12. Système de distribution selon l'une quelconque des revendications 1 à 11, **caractérisé en ce qu'**un clapet de non-retour à bille est disposé après au moins une ouverture de sortie (3, 4).

13. Système de distribution selon l'une quelconque des revendications 1 à 12, **caractérisé en ce qu'**un bâtonnet entouré d'ouate est disposé après au moins une ouverture de sortie (3, 4).

14. Système de distribution selon l'une quelconque des revendications 1 à 13, **caractérisé en ce qu'**un peigne est disposé après au moins une ouverture de sortie (3, 4).

15. Système de distribution selon l'une quelconque des revendications 1 à 14, **caractérisé en ce que** les récipients (1, 2) sont réalisés de manière identique.

16. Système de distribution selon l'une quelconque des revendications 1 à 15, **caractérisé en ce qu'**au moins un des agents modifiant la couleur des cheveux contient au moins une substance d'entretien.

17. Système de distribution selon l'une quelconque des revendications 1 à 16, **caractérisé en ce qu'**au moins un des agents modifiant la couleur des cheveux contient au moins un agent tensioactif.

18. Procédé pour l'application de petites mèches, **caractérisé en ce qu'**on applique sur les fibres de manière simultanée au moins deux agents différents modifiant la couleur des cheveux au moyen d'un système de distribution selon l'une quelconque des revendications 1 à 17, on les y laisse pendant un certain laps de temps et on rince ensuite les fibres en profondeur.

19. Procédé selon la revendication 18, **caractérisé en ce que** les fibres sont mises en forme avant l'application des agents modifiant la couleur des cheveux.
